# EUROPEAN PATENT APPLICATION

(11) **EP 4 726 394 A1**
(43) Date of publication of application: **15.04.2026**
(21) Application number: 25208337.3
(22) Date of filing: 13.10.2025
(51) Int. Cl.: G01N 33/72, G16H 50/20

(54) **AUTOMATIC REVIEW METHOD FOR GLYCATED HEMOGLOBIN, SAMPLE ANALYSIS DEVICE, AND SAMPLE ANALYSIS SYSTEM**

(30) Priority: 11.10.2024 CN 202411423786
(71) Applicant: Shenzhen Mindray Bio-Medical Electronics Co., Ltd., Shenzhen, Guangdong 518057 (CN)
(72) Inventor: WU, Junbin, Shenzhen, 518057 (CN); WANG, Xian, Shenzhen, 518057 (CN); LI, Jin, Shenzhen, 518057 (CN)
(74) Representative: KIPA AB

(57) **Abstract**

An automatic review method for glycated hemoglobin, a sample analysis device, and a sample analysis system are provided. The method includes: obtaining basic information for a subject, and obtaining a test value related to blood glucose and a test value related to red blood cell for the subject; calculating an estimated value of glycated hemoglobin for the subject based on the basic information, the test value related to blood glucose and the test value related to red blood cell; and performing an automatic review on the test value of glycated hemoglobin for the subject based on the estimated value of glycated hemoglobin for the subject. The disclosure provides an automatic review solution for glycated hemoglobin, which may reduce workload of a reviewer, allowing the reviewer to focus on reviewing a small number of glycated hemoglobin test values that do not pass the automatic review and are suspected to be problematic.

## Description

### TECHNICAL FIELD

The disclosure relates to the field of in vitro diagnosis, and in particular, to an automatic review method for glycated hemoglobin, a sample analysis device, and a sample analysis system.

### BACKGROUND

Diabetes mellitus is caused by insufficient insulin secretion or ineffective insulin action, accompanied by metabolic disorders of carbohydrates, proteins, fats, water, and electrolytes. As a good indicator of long-term blood glucose control in diabetic patients, glycated hemoglobin (HbA1c) plays an important role not only in diagnosis and screening of diabetes, but also in diagnosis and assessment of some complications of diabetes. Glycated hemoglobin (HbA1c) test is not subject to time limits, may reflect an average blood glucose level of a patient over two to three months before blood collection, and is currently internationally recognized as a "gold standard" for testing serum control in diabetic patients. The glycated hemoglobin (HbA1c) test is influenced by factors such as test methods, human factors, and the standardization construction of laboratories, which leads to deviations in the test results, and leads to the test results often being inconsistent with the actual situation. Currently, clinical research on the glycated hemoglobin (HbA1c) test primarily focuses on testing methods and influencing factors, and there is no automatic review for the glycated hemoglobin (HbA1c) test. Currently, it still relies on manual review by medical personnel, which imposes significant review pressure on the medical personnel.

### SUMMARY

In order to solve the above problem, the disclosure provides an automatic review method for glycated hemoglobin, a sample analysis device and a sample analysis system, which will be described in detail below.

According to a first aspect, an embodiment provides an automatic review method for glycated hemoglobin. The method includes:
testing a sample of a subject to obtain a test value of glycated hemoglobin for the subject;
obtaining basic information of first type for the subject, where the basic information of first type is quantifiable information that represents personal characteristic of the subject;
obtaining a test value related to blood glucose for the subject and a test value related to red blood cell for the subject;
calculating an estimated value for glycated hemoglobin of the subject based on the first-type basic information, the test value related to blood glucose and the test value related to red blood cell; and
performing an automatic review on the test value of glycated hemoglobin for the subject based on the estimated value of glycated hemoglobin for the subject.

In an embodiment, the test value related to blood glucose includes: a test value that reflects a related substance involved in blood glucose metabolism and/or a test value that reflects a substance related to blood glucose concentration;
preferably, the test value that reflects the related substance involved in blood glucose metabolism includes at least one of followings: a related test value that reflects insulin, and a related test value that reflects a metabolite of insulin; preferably, the related test value that reflects insulin includes at least one of followings: a test value of glutamate decarboxylase antibody, a test value of insulin autoantibody, a test value of islet cell antibody, a test value of islet cell antigen 2 antibody, and a test value of zinc transporter 8 antibody; the related test value that reflects the metabolite of insulin includes at least one of followings: a test value of serum C-peptide, a test value of adiponectin, and a test value of fetuin;
preferably, the test value that reflects the substance related to blood glucose concentration includes at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin; preferably, the test value of blood glucose includes at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin includes at least one of followings: a test value of total bilirubin, a test value of direct bilirubin, and a test value of indirect bilirubin.

In an embodiment, the test value related to red blood cell includes at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.

In an embodiment, the basic information of first type includes at least one of followings: age, height, weight, and body mass index.

In an embodiment, calculating an estimated value of glycated hemoglobin for the subject based on the basic information of first type, the test value related to blood glucose and the test value related to red blood cell, includes:
inputting the basic information of first type, the test value related to blood glucose and the test value related to red blood cell into a glycated hemoglobin estimation model, so as to obtain the estimated value of glycated hemoglobin for the subject through calculation, where the glycated hemoglobin estimation model is configured to use the basic information of first type, the test value related to blood glucose and the test value related to red blood cell as input data, and perform calculation to output the estimated value of glycated hemoglobin for the subject;
where the glycated hemoglobin estimation model is obtained through training by using a training set, where data of the training set includes first data and second data, the first data is labeled with the second data, the first data includes first-type basic information, test values related to blood glucose and test values related to red blood cell for other subjects, and the second data includes test values of glycated hemoglobin for said subjects.

In an embodiment, the glycated hemoglobin estimation model includes: a convolutional neural network-based model, a recurrent neural network-based model, a generative adversarial neural network-based model, an attention neural network-based model, or a fully connected network-based model.

In an embodiment, calculating an estimated value of glycated hemoglobin for the subject based on the basic information of first type, the test value related to blood glucose and the test value related to red blood cell, includes:
obtaining a function relationship between the basic information of first type, the test value related to blood glucose, the test value related to red blood cell and the estimated value of glycated hemoglobin; and
obtaining the estimated value of glycated hemoglobin for the subject based on the function relationship, the basic information of first type for the subject, the test value related to blood glucose for the subject and the test value related to red blood cell for the subject.

In an embodiment, the function relationship includes a first function formula; obtaining the estimated value of glycated hemoglobin for the subject based on the function relationship, the basic information of first type for the subject, the test value related to blood glucose for the subject and the test value related to red blood cell for the subject, includes:
inputting the basic information of first type for the subject, the test value related to blood glucose for the subject and the test value related to red blood cell for the subject as input values into the first function formula, so as to obtain the estimated value of glycated hemoglobin for the subject through calculation.

In an embodiment, the first function formula includes a function formula that preforms linear weighting on a plurality of input values.

In an embodiment, the basic information of first type is age, the test value related to blood glucose is a test value of blood glucose, the test value related to red blood cell is a test value of red blood cell count, and the first function formula is: HbA1c' = a1 + a2*Age + a3*GLU + a4*RBC, where HbA1c' represents the estimated value of glycated hemoglobin, Age represents the age, GLU represents the test value of blood glucose, RBC represents the test value of red blood cell count, and a1, a2, a3, and a4 represent constants; preferably, a1 ranges from 1.6 to 2.6, a2 ranges from 0.01 to 0.02, a3 ranges from 0.4 to 0.5, and a4 ranges from 0.07 to 0.24, or
the basic information of first type is age, the test value related to blood glucose includes a test value of fasting blood glucose, a test value of 2-hour postprandial blood glucose and a test value of bilirubin, the test value related to red blood cell is a test value of red blood cell count, and the first function formula is: HbA1c' = b1 + b2*Age + b3*GLU 1 + b4*G120 + bS/IB² + b6*RBC, where HbA1c' represents the estimated value of glycated hemoglobin, Age represents the age, GLU1 represents the test value of fasting blood glucose, G120 represents the test value of 2-hour postprandial blood glucose, IB represents the test value of bilirubin, RBC represents the test value of red blood cell count, and b1, b2, b3, b4, b5, and b6 represent constants; preferably, b1 ranges from 1.6 to 2.5, b2 ranges from 0.001 to 0.009, b3 ranges from 0.24 to 0.3, b4 ranges from 0.09 to 0.13, b5 ranges from 2.5 to 10.9, and b6 ranges from 0.13 to 0.29; or
the basic information of first type is age, the test value related to blood glucose includes a test value of blood glucose and a test value of bilirubin, the test value related to red blood cell is a test value of red blood cell count, and the first function formula is: HbA1c' = c1 + c2*Age + c3*GLU + c4/IB² + c5*RBC, where HbA1c' represents the estimated value of glycated hemoglobin, Age represents the age, GLU represents the test value of blood glucose, IB represents the test value of bilirubin, RBC represents the test value of red blood cell count, and c1, c2, c3, c4, and c5 represent constants; preferably, c1 ranges from 1.2 to 2.3, c2 ranges from 0.01 to 0.02, c3 ranges from 0.4 to 0.47, c4 ranges from 2.8 to 12.97, and c5 ranges from 0.1 to 0.29.

In an embodiment, performing an automatic review on the test value of glycated hemoglobin for the subject based on the estimated value of glycated hemoglobin for the subject, includes: calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate.

In an embodiment, calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate, includes:
calculating a confidence interval of glycated hemoglobin for the subject based on the estimated value of glycated hemoglobin for the subject; and
determining that the test value of glycated hemoglobin for the subject is accurate when the test value of glycated hemoglobin for the subject falls within the confidence interval of glycated hemoglobin for the subject.

In an embodiment, calculating a confidence interval of glycated hemoglobin for the subject based on the estimated value of glycated hemoglobin for the subject includes: obtaining the confidence interval of glycated hemoglobin for the subject through calculation based on the estimated value of glycated hemoglobin for the subject and a second function formula.

In an embodiment, the second function formula includes: CI1 = HbA1c' - D1; CI2 = HbA1c' + D2, where HbA1c' represents the estimated value of glycated hemoglobin, and D1 and D2 represent constants;
the confidence interval CI for glycated hemoglobin of the subject satisfies: CI = [CI1, CI2], where CI1 represents a left endpoint of the confidence interval, CI2 represents a right endpoint of the confidence interval, and [CI1, CI2] represents an interval constituted by the two endpoints.

In an embodiment, D1 is determined based on a standard deviation of glycated hemoglobin of a sample library and a preset confidence level; and/or, D2 is determined based on a standard deviation of glycated hemoglobin of a sample library and a preset confidence level, where the sample library includes test values of glycated hemoglobin of a plurality of samples; preferably, D1 and D2 are equal.

In an embodiment, the automatic review includes: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.

In an embodiment, the method further includes: prompting a manual review when the basic information of first type, the test value related to blood glucose, and/or the test value related to red blood cell cannot be obtained.

According to a second aspect, an embodiment provides an automatic review method for glycated hemoglobin. The method includes:
testing a sample of a subject to obtain a test value of glycated hemoglobin for the subject;
obtaining basic information for the subject;
obtaining a test value related to blood glucose for the subject and a test value related to red blood cell for the subject;
calculating an estimated value of glycated hemoglobin for the subject based on the basic information, the test value related to blood glucose and the test value related to red blood cell; and
performing an automatic review on the test value of glycated hemoglobin for the subject based on the estimated value of glycated hemoglobin for the subject.

In an embodiment, the basic information includes at least one of followings: age, height, weight, body mass index, history of diabetes, medication status, and sample source department.

In an embodiment, calculating an estimated value of glycated hemoglobin for the subject based on the basic information, the test value related to blood glucose and the test value related to red blood cell, includes:
inputting the basic information, the test value related to blood glucose and the test value related to red blood cell into a glycated hemoglobin estimation model, so as to obtain the estimated value of glycated hemoglobin for the subject through calculation, where the glycated hemoglobin estimation model is configured to use the basic information, the test value related to blood glucose and the test value related to red blood cell as input data, and perform calculation to output the estimated value of glycated hemoglobin for the subject; the glycated hemoglobin estimation model is obtained through training by using a training set, where data of the training set includes first data and second data, the first data is labeled with the second data, the first data includes basic information, test values related to blood glucose and test values related to red blood cell for other subjects, and the second data includes test values of glycated hemoglobin for said subjects;
   or
obtaining a function relationship between the basic information, the test value related to blood glucose, the test value related to red blood cell, and the estimated value of glycated hemoglobin; and obtaining the estimated value of glycated hemoglobin for the subject based on the function relationship, the basic information for the subject, the test value related to blood glucose for the subject and the test value related to red blood cell for the subject.

In an embodiment, the function relationship includes a first function formula; obtaining the estimated value of glycated hemoglobin for the subject based on the function relationship, the basic information for the subject, the test value related to blood glucose for the subject and the test value related to red blood cell for the subject, includes:
inputting the basic information for the subject, the test value related to blood glucose for the subject and the test value related to red blood cell for the subject as input values into the first function formula, so as to obtain the estimated value of glycated hemoglobin for the subject through calculation.

In an embodiment, the first function formula includes a function formula that preforms linear weighting on a plurality of input values.

In an embodiment, the automatic review includes: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.

According to a third aspect, an embodiment provides an automatic review method for glycated hemoglobin. The method includes:
testing a sample of a subject to obtain a test value of glycated hemoglobin for the subject;
obtaining basic information of first type for the subject, where the basic information of first type is quantifiable information that represents personal characteristic of the subject;
obtaining an in vitro diagnostic test value associated with glycated hemoglobin for the subject;
calculating an estimated value of glycated hemoglobin for the subject based on the basic information of first type and the in vitro diagnostic test value associated with glycated hemoglobin; and
performing an automatic review on the test value of glycated hemoglobin for the subject based on the estimated value of glycated hemoglobin for the subject.

In an embodiment, the in vitro diagnostic test value associated with glycated hemoglobin includes at least one of followings: a test value related to blood glucose, and a test value related to red blood cell.

In an embodiment, the test value related to blood glucose includes: a test value that reflects a related substance involved in blood glucose metabolism and/or a test value that reflects a substance related to blood glucose concentration;
preferably, the test value that reflects the related substance involved in blood glucose metabolism includes at least one of followings: a related test value that reflects insulin, and a related test value that reflects a metabolite of insulin; preferably, the related test value that reflects insulin includes at least one of followings: a test value of glutamate decarboxylase antibody, a test value of insulin autoantibody, a test value of islet cell antibody, a test value of islet cell antigen 2 antibody, and a test value of zinc transporter 8 antibody; the related test value that reflects the metabolite of insulin includes at least one of followings: a test value of serum C-peptide, a test value of adiponectin, and a test value of fetuin;
preferably, the test value that reflects the substance related to blood glucose concentration includes at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin; preferably, the test value of blood glucose includes at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin includes at least one of followings: a test value of total bilirubin, a test value of direct bilirubin, and a test value of indirect bilirubin;
   and/or
the test value related to red blood cell includes at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.

In an embodiment, the in vitro diagnostic test value associated with glycated hemoglobin includes at least one of followings: a biochemical test value associated with glycated hemoglobin, a blood routine test value associated with glycated hemoglobin, an immunological test value associated with glycated hemoglobin, and a mass spectrometry test value associated with glycated hemoglobin.

In an embodiment, the biochemical test value associated with glycated hemoglobin includes at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, and a test value of bilirubin; preferably, the test value of blood glucose includes at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin includes at least one of followings: a test value of total bilirubin, a test value of direct bilirubin, and a test value of indirect bilirubin;
and/or
the blood routine test value associated with glycated hemoglobin includes at least one of followings: a test value of lactic acid, and a test value related to red blood cell; preferably, the test value related to red blood cell includes at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width;
   and/or
the immunological test value associated with glycated hemoglobin includes at least one of followings: a test value of glutamate decarboxylase antibody, a test value of insulin autoantibody, a test value of islet cell antibody, a test value of islet cell antigen 2 antibody, a test value of zinc transporter 8 antibody, a test value of serum C-peptide, a test value of adiponectin, and a test value of fetuin;
   and/or
the mass spectrometry test value associated with glycated hemoglobin includes at least one of followings: a test value of 1-anhydroglucitol, and a test value of 5-anhydroglucitol.

In an embodiment, calculating an estimated value of glycated hemoglobin for the subject based on the basic information of first type and the in vitro diagnostic test value associated with glycated hemoglobin, includes:
inputting the basic information of first type and the in vitro diagnostic test value associated with glycated hemoglobin into a glycated hemoglobin estimation model, so as to obtain the estimated value of glycated hemoglobin for the subject through calculation, where the glycated hemoglobin estimation model is configured to use the basic information of first type and the in vitro diagnostic test value associated with glycated hemoglobin as input data, and perform calculation to output the estimated value of glycated hemoglobin for the subject; the glycated hemoglobin estimation model is obtained through training by using a training set, where data of the training set includes first data and second data, the first data is labeled with the second data, the first data includes basic information of first type and in vitro diagnostic test values associated with glycated hemoglobin for other subjects, and the second data includes test values of glycated hemoglobin for said subjects;
   or
obtaining a function relationship between the basic information of first type, the in vitro diagnostic test value associated with glycated hemoglobin, and the estimated value of glycated hemoglobin; obtaining the estimated value of glycated hemoglobin for the subject based on the function relationship, the basic information of first type for the subject and the in vitro diagnostic test value associated with glycated hemoglobin for the subject.

In an embodiment, the function relationship includes a first function formula; obtaining the estimated value of glycated hemoglobin for the subject based on the function relationship, the basic information of first type for the subject and the in vitro diagnostic test value associated with glycated hemoglobin for the subject, includes:
inputting the basic information of first type for the subject and the in vitro diagnostic test value associated with glycated hemoglobin for the subject as input values into the first function formula, so as to obtain the estimated value of glycated hemoglobin for the subject through calculation.

In an embodiment, the automatic review includes: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.

According to a fourth aspect, an embodiment provides an automatic review method for glycated hemoglobin. The method includes:
testing a sample of a subject to obtain a test value of glycated hemoglobin for the subject;
obtaining basic information for the subject;
obtaining an in vitro diagnostic test value associated with glycated hemoglobin for the subject;
calculating an estimated value of glycated hemoglobin for the subject based on the basic information and the in vitro diagnostic test value associated with glycated hemoglobin; and
performing automatic review on the test value of glycated hemoglobin for the subject based on the estimated value of glycated hemoglobin for the subject.

In an embodiment, calculating an estimated value of glycated hemoglobin for the subject based on the basic information and the in vitro diagnostic test value associated with glycated hemoglobin, includes:
inputting the basic information and the in vitro diagnostic test value associated with glycated hemoglobin into a glycated hemoglobin estimation model, so as to obtain the estimated value of glycated hemoglobin for the subject through calculation, where the glycated hemoglobin estimation model is configured to use the basic information the in vitro diagnostic test value associated with glycated hemoglobin and as input data, and perform calculation to output the estimated value of glycated hemoglobin for the subject; the glycated hemoglobin estimation model is obtained through training by using a training set, where data of the training set includes first data and second data, the first data is labeled with the second data, the first data includes basic information and in vitro diagnostic test values associated with glycated hemoglobin for other subjects, and the second data includes test values of glycated hemoglobin for said subjects;
   or
obtaining a function relationship between the basic information, the in vitro diagnostic test value associated with glycated hemoglobin, and the estimated value of glycated hemoglobin; obtaining the estimated value of glycated hemoglobin for the subject based on the function relationship, the basic information and the in vitro diagnostic test value associated with glycated hemoglobin.

In an embodiment, the function relationship includes a first function formula; obtaining the estimated value of glycated hemoglobin for the subject based on the function relationship, the basic information and the in vitro diagnostic test value associated with glycated hemoglobin, includes:
inputting the basic information for the subject, and the in vitro diagnostic test value associated with glycated hemoglobin as input values into the first function formula, so as to obtain the estimated value of glycated hemoglobin for the subject through calculation.

In an embodiment, the automatic review includes: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.

According to a fifth aspect, an embodiment provides an automatic review method for glycated hemoglobin. The method includes:
testing a sample of a subject to obtain a test value of glycated hemoglobin for the subject;
obtaining a confidence interval of glycated hemoglobin for the subject, where the confidence interval of glycated hemoglobin for the subject is configured to evaluate the test value of glycated hemoglobin for the subject; and
performing automatic review on the test value of glycated hemoglobin for the subject based on the confidence interval of glycated hemoglobin for the subject.

In an embodiment, the confidence interval of glycated hemoglobin for the subject is obtained through calculation based on an estimated value of glycated hemoglobin for the subject; or, the confidence interval of glycated hemoglobin for the subject is obtained through calculation based on a mean and a standard deviation of test values of glycated hemoglobin of a plurality of samples in a sample library.

In an embodiment, the estimated value of glycated hemoglobin for the subject is obtained through calculation based on basic information and an in vitro diagnostic test value associated with glycated hemoglobin.

In an embodiment, the automatic review includes: outputting the test value of glycated hemoglobin for the subject when the test value and the estimated value of glycated hemoglobin for the subject satisfy a preset relationship; or prompting manual review when the test value and the estimated value of glycated hemoglobin for the subject do not satisfy the preset relationship.

According to a sixth aspect, an embodiment provides an automatic review method for glycated hemoglobin. The method includes:
testing a sample of a subject to obtain a test value of glycated hemoglobin for the subject;
obtaining information of the subject, where the information includes basic information or an in vitro diagnostic test value associated with glycated hemoglobin for the subject;
calculating an estimated value of glycated hemoglobin for the subject based on the information of the subject; and
performing automatic review on the test value of glycated hemoglobin for the subject based on the estimated value of glycated hemoglobin for the subject.

In an embodiment, calculating an estimated value of glycated hemoglobin for the subject based on the information of the subject, includes:
inputting the information of the subject into a glycated hemoglobin estimation model, so as to obtain the estimated value of glycated hemoglobin for the subject through calculation, where the glycated hemoglobin estimation model is configured to use the information of the subject as input data, and perform calculation to output the estimated value of glycated hemoglobin for the subject; the glycated hemoglobin estimation model is obtained through training by using a training set, where data of the training set includes first data and second data, the first data is labeled with the second data, the first data includes information of other subjects, and the second data includes test values of glycated hemoglobin for said subjects;
   or
obtaining a function relationship between the information and the estimated value of glycated hemoglobin; and obtaining the estimated value of glycated hemoglobin for the subject based on the function relationship and the information of the subject.

In an embodiment, the function relationship includes a first function formula; obtaining the estimated value of glycated hemoglobin for the subject based on the function relationship and the information of the subject, includes:
inputting the information of the subject as an input value into the first function formula, so as to obtain the estimated value of glycated hemoglobin for the subject through calculation.

In an embodiment, the automatic review includes: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.

According to a seventh aspect, an embodiment provides a sample analysis device. The device includes:
a measurement component configured to test a sample to obtain a test value for an item;
a sample component configured to provide a sample to the measurement component;
a reagent component configured to provide a reagent to the measurement component; and
a processor configured to perform the automatic review method for glycated hemoglobin described in any of the embodiments herein.

According to an eighth aspect, an embodiment provides a sample analysis system. The system includes: a processor and a plurality of cascaded analysis devices, where each of the analysis devices is configured to test a sample to obtain a test value for an item, and the processor is configured to perform the automatic review method for glycated hemoglobin described in any of the embodiments herein.

The automatic review method for glycated hemoglobin, the sample analysis device, and the sample analysis system according to the above-described embodiments provide an automatic review solution for glycated hemoglobin, which can reduce workload of a reviewer, allowing the reviewer to focus on reviewing a small number of glycated hemoglobin test values that do not pass the automatic review and are suspected to be problematic.

### BRIEF DESCRIPTION OF THE DRAWINGS

FIG. 1 is a schematic structural diagram of a sample analysis device according to an embodiment;
FIG. 2 is a schematic structural diagram of a sample analysis device according to an embodiment;
FIG. 3 is a schematic structural diagram of a sample analysis system according to an embodiment;
FIG. 4 is a schematic structural diagram of a sample analysis system according to an embodiment;
FIG. 5 is a schematic structural diagram of a sample analysis system according to an embodiment;
FIG. 6 is a schematic structural diagram of a pre-processing module according to an embodiment;
FIG. 7 is a schematic structural diagram of a post-processing module according to an embodiment;
FIG. 8 is a schematic structural diagram of a sample analysis system according to an embodiment;
FIG. 9 is a schematic structural diagram of an automatic review device for glycated hemoglobin according to an embodiment;
FIG. 10 is a flowchart of an automatic review method for glycated hemoglobin according to an embodiment;
FIG. 11 is a flowchart of calculating an estimated value for glycated hemoglobin of a subject according to an embodiment;
FIG. 12 is a flowchart of performing automatic review on a test value for glycated hemoglobin of a subject based on an estimated value of glycated hemoglobin for the subject according to an embodiment; and
FIG. 13 is a flowchart of an automatic review method for glycated hemoglobin according to an embodiment.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, a detailed description of the involved operations is not necessary, and the involved operations can be thoroughly understood according to the description in the specification and general technical knowledge of the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, various orders in the specification and the accompanying drawings are merely for the purpose of a clear description of a certain embodiment and are not meant to be necessary orders unless it is otherwise stated that a certain order must be followed.

The serial numbers themselves for the components herein, for example, "first", "second", etc. are merely used to distinguish the described objects, and do not have any sequential or technical meaning. Moreover, as used in the disclosure, "connection" or "coupling", unless otherwise stated, includes both direct and indirect connections (couplings).

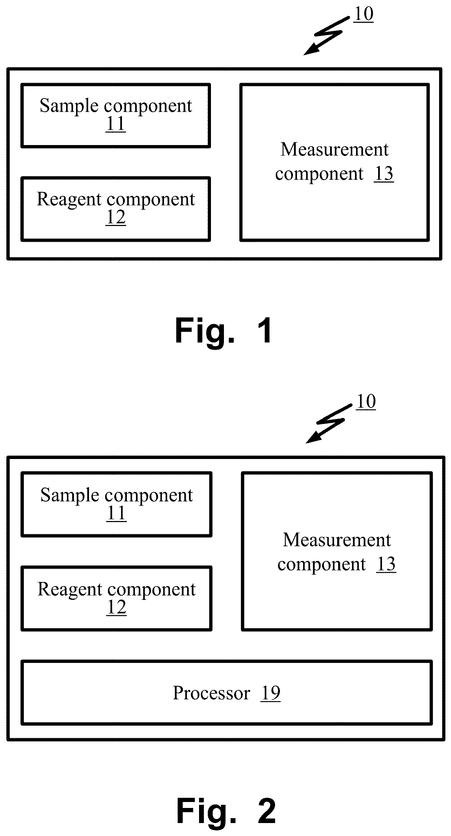

Referring to FIG. 1, a sample analysis device 10 is disclosed in some embodiments. The sample analysis device 10, which may also be referred to as an analysis device 10, is capable of testing a sample for one or more items to obtain a test value for a corresponding item. The sample may include blood, body fluid or even saliva, etc., depending on different types of the sample analysis device 10 and different items. The items may include one or more of followings: a test item for glutamate decarboxylase antibody, a test item for insulin autoantibody, a test item for islet cell antibody, a test item for islet cell antigen 2 antibody, a test item for zinc transporter 8 antibody, a test item for serum C-peptide, a test item for adiponectin, a test item for fetuin, a test item for blood glucose, a test item for 1-anhydroglucitol, a test item for 5-anhydroglucitol, a test item for lactic acid, a test item for glycated serum protein, a test item for fructosamine, a test item for glycated serum albumin, a test item for advanced glycation end products, a test item for bilirubin, a test item for fasting blood glucose, a test item for 1-hour postprandial blood glucose, a test item for 2-hour postprandial blood glucose, a test item for 3-hour postprandial blood glucose, a test item for total bilirubin, a test item for direct bilirubin, a test item for indirect bilirubin, a test item for red blood cell count, a test item for hemoglobin content, a test item for mean corpuscular volume, a test item for mean corpuscular hemoglobin, a test item for mean corpuscular hemoglobin concentration, a test item for hematocrit, and a test item for red blood cell distribution width. Specific test items that the sample analysis device 10 can perform may be designed according to actual needs.

In some embodiments, the sample analysis device 10 may be any one of the following types: a biochemical analysis device, a blood analysis device, an immunological analysis device, and a mass spectrometry analysis device.

In some embodiments, the sample analysis device 10 may include a sample component 11, a reagent component 12, and a measurement component 13. The measurement component 13 is configured to test a sample to obtain a test value for an item, the sample component 11 is configured to provide a sample to the measurement component 13, and the reagent component 12 is configured to provide a reagent to the measurement component 13. The detailed description is given below.

In some embodiments, the sample component 11 is configured to hold a sample to be tested and may provide the sample to the measurement component 13. In some embodiments, the sample component 11 is capable of holding and even dispatching the sample. The sample may be held and dispatched in a sample rack, and the sample component 11 accordingly has a related structure for holding and dispatching the sample rack. For example, the sample component 11 includes a track-based sample feeding structure, through which the sample rack is dispatched. The sample may also be held and transported in a sample tube, and the sample component 11 accordingly has a related structure for holding and transporting the sample tube. For example, the sample component 11 includes a rotatable disk structure, which is provided with a sample position for placing the sample tube. In addition, the sample component 11 may further have a dispensing structure for dispensing the sample to implement dispensing of the sample to be tested to the measurement component 13.

In some embodiments, the reagent component 12 is configured to hold a reagent, and provide the reagent to the measurement component 13. In some embodiments, the reagent component 12 includes a rotatable disk structure, which is provided with a reagent position for placing a reagent container. In addition, the reagent component 12 may further have a dispensing structure for dispensing the reagent to implement dispensing of the reagent to the measurement component 13. In some embodiments, the reagent component 12 may alternatively include a container that holds the reagent, and a fluid circuit structure, through which the reagent is dispensed from the container to the measurement component 13.

In some embodiments, the measurement component 13 is configured to test a sample to obtain a test value for an item. That is, the measurement component tests a test sample formed by the sample and the reagent to obtain test data, and obtains the test value based on the test data. In some embodiments, the measurement component 13 may include a structure for the sample and the reagent to mix or react, and a structure for testing the test sample formed by the sample and the reagent.

Referring to FIG. 2, in some embodiments, the sample analysis device 10 may further include a processor 19.

In some embodiments, the processor 19 is configured to control the sample component 11, the reagent component 12 and the measurement component 13 to cooperate to complete testing of the sample. For example, the processor is configured to control the sample component 11 to provide the sample to the measurement component 13, control the reagent component 12 to provide the reagent to the measurement component 13, and control the measurement component 13 to test the test sample formed by the sample and the reagent to obtain a test value for an item.

In some embodiments, the processor 19 is configured to execute each computer application program in a non-transitory computer-readable storage medium so as to perform corresponding steps and methods. For example, the processor 19 may be implemented by software, hardware, firmware, or a combination thereof, and may use at least one of a circuit, one or more application specific integrated circuits (ASIC), a digital signal processor (DSP), a digital signal processing device (DSPD), a programmable logic device (PLD), a field programmable gate array (FPGA), a central processing unit (CPU), a controller, a microcontroller, and a microprocessor, such that the processor 19 may perform some or all of the steps or any combination of the steps in the automatic review method according to the embodiments of the disclosure, which will be described in detail below.

The foregoing is some descriptions of the sample analysis device 10.

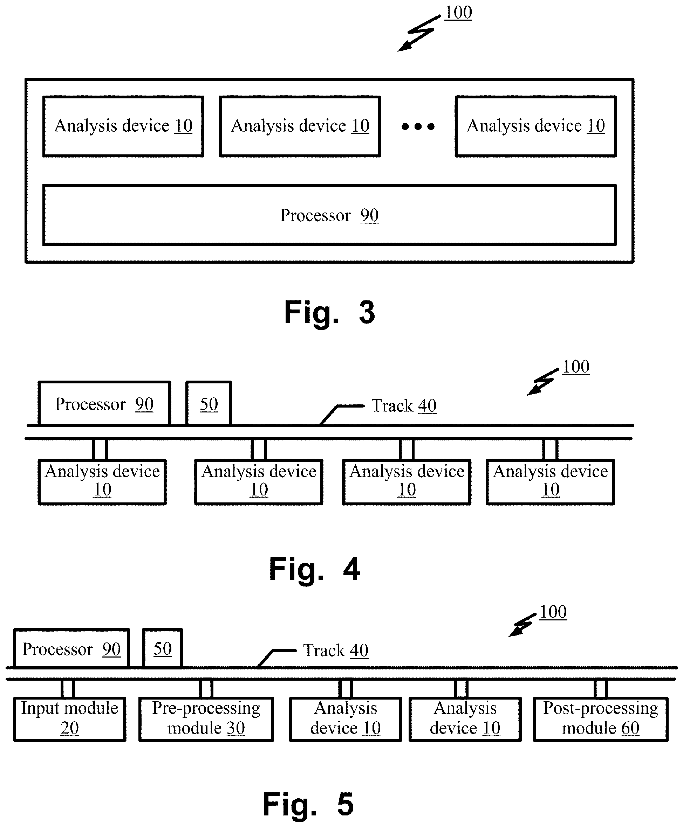

Referring to FIG. 3, a sample analysis system 100 is disclosed in some embodiments. The sample analysis system 100 includes a plurality of cascaded sample analysis devices 10. As described above, each of the sample analysis devices 10 may also be referred to as an analysis device 10, and each of the analysis devices 10 is configured to test a sample to obtain a test value for an item. An analysis device 10 may include a sample component 11, a reagent component 12, a measurement component 13, etc., which are described above and are not described herein again. Multiple analysis devices 10 included in the sample analysis system 100 may be analysis devices 10 of a same type (i.e., configured for same test items) or may be analysis devices 10 of different types (i.e., configured for different test items). For example, each of the analysis devices 10 may be any one of a biochemical analysis device, a blood analysis device, an immunoassay device, and a mass spectrometry device. That is, the sample analysis system 100 may include analysis devices 10 of any one type or more types selected from a biochemical analysis device, a blood analysis device, an immunological analysis device and a mass spectrometry analysis device.

Referring to FIG. 4, in some embodiments, the sample analysis system 100 may include a track 40 and a dispatching apparatus 50. Multiple analysis devices 10 are cascaded by the track 40, that is, the track 40 cascades the analysis devices 10 so that a sample reaches any one of the analysis devices 10 for testing through the track 40. The dispatching apparatus 50 is configured to dispatch the sample through the track 40, for example, dispatch the sample from one analysis device 10 to another analysis device 10. It should be noted that FIG. 4 shows four analysis devices 10, which is for illustration only and is not intended to limit the sample analysis system 100 to only including four analysis devices 10. It may be understood that the sample analysis system 100 may include fewer or more analysis devices 10, depending on design requirements.

Referring to FIG. 5, in some embodiments, the sample analysis system 100 may further include one or more of an input module 20, a pre-processing module 30, a post-processing module 60, or the like. In such an example, these modules are connected by the track 40. It should be noted that FIG. 5 shows two analysis devices 10, which is for illustration only and is not intended to limit the sample analysis system 100 to only including two analysis devices 10. It may be understood that the sample analysis system 100 may include more analysis devices 10 depending on design requirements.

In some embodiments, the input module 20 may be configured to receive a sample to be tested that is placed by a user. In some embodiments, the input module 20 may further obtain identification information of the sample to be tested. The user may place the sample to be tested in the input module 20, and the input module 20 may, for example, scan a label, such as a bar code or a two-dimensional code, on the sample to be tested, by using a scanning apparatus, so as to obtain identification information of the sample. The identification information may include, for example, a sample ID, a sample type, sample source information, etc.

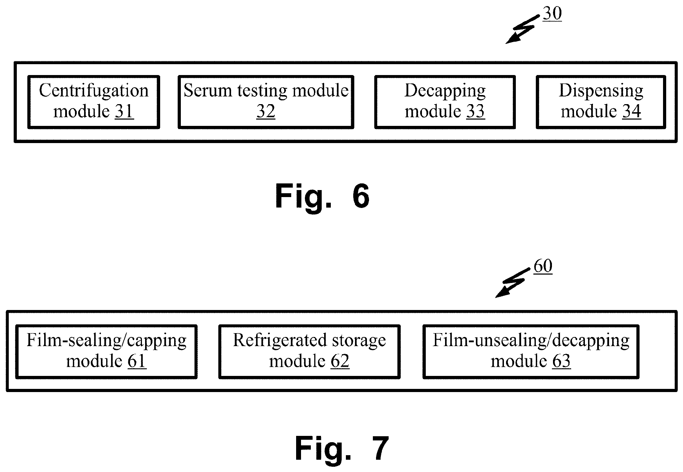

In some embodiments, the pre-processing module 30 is configured to perform pre-processing on the sample to be tested that is received by the input module 20. Generally, after the user places the sample in the input module 20, the input module 20 scans the sample, the dispatching apparatus 50 then dispatches the sample to the pre-processing module 30 for pre-processing, and the pre-processed sample is then dispatched from the pre-processing module 30 to a corresponding analysis device 10 for testing. In an embodiment, referring to FIG. 6, the pre-processing module 30 may include one or more of a centrifugation module 31, a serum testing module 32, a decapping module 33, and a dispensing module 34. The centrifugation module 31 is configured to centrifuge a sample to be centrifuged. One or more centrifugation modules 31 may be provided. The serum testing module 32 is configured to test whether serum in the sample is sufficient and/or whether serum in a sample is qualified, so as to determine whether the centrifuged sample can be used for subsequent measurement. The decapping module 33 is configured to decap the centrifuged sample. It may be understood that capping, film sealing, decapping, and film unsealing of a sample herein refer to capping, film sealing, decapping, and film unsealing of a sample tube that contains the sample. Generally, a sample needs to be decapped after being centrifuged, so that the dispensing module 34 or the analysis device subsequently performs sample dispensing or sample aspiration. The dispensing module 34 is configured to dispense the sample, for example, to divide the sample into a plurality of parts and deliver the plurality of parts to different analysis devices 10 for testing respectively. A general pre-processing procedure of the pre-processing module 30 is as follows: the centrifugation module 31 receives and centrifuges a sample dispatched by the input module 20; the serum testing module 32 tests serum in the centrifuged sample and determines whether the sample can be used for subsequent measurement, and if the serum is insufficient or unqualified, the sample cannot be used for subsequent measurement; and if the serum test of the sample is passed, the sample is dispatched to the decapping module 33, and the decapping module 33 decaps the sample; if a dispensing module 34 is provided, the dispensing module 34 dispenses the decapped sample and dispatches the dispensed sample to the corresponding analysis device 10 for testing, and if no dispensing module 34 is provided, the sample is dispatched from the decapping module 33 to the corresponding analysis device 10 for testing. It should be noted that the pre-processing module 30 is not mandatory.

The post-processing module 60 is configured to perform post-processing on the sample. In an embodiment, referring to FIG. 7, the post-processing module 60 includes one or more of a film-sealing/capping module 61, a refrigerated storage module 62 and a film-unsealing/decapping module 63. The film-sealing/capping module 61 is configured to film-seal or cap the sample. The refrigerated storage module 62 is configured to store the sample. The film-unsealing/decapping module 63 is configured to film-unseal or decap the sample. A general post-processing procedure of the post-processing module 60 is as follows: after the sample is aspirated by all the analysis devices 10 through which the sample are required to be tested, the sample is dispatched to the film-sealing/capping module 61, the film-sealing/capping module 61 film-seals or caps the tested sample, and then the sample is dispatched to the refrigerated storage module 62 for storage; and if the sample needs to be retested, the sample is dispatched out of the refrigerated storage module 62, film-unsealed or decapped in the film-unsealing/decapping module 63, and then dispatched to a corresponding analysis device 10 for retest. It should be noted that the post-processing module 60 is not mandatory.

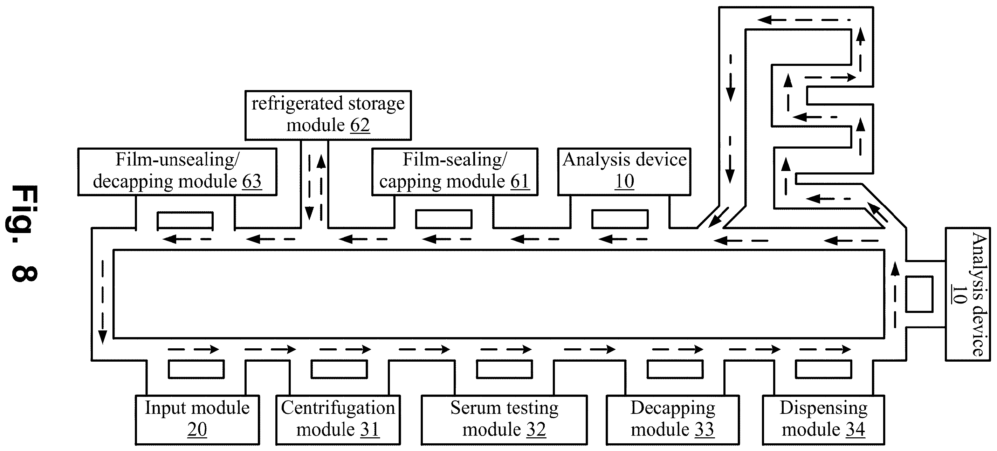

FIG. 8 is a schematic structural diagram of the sample analysis system 100. As shown in FIG. 8, each analysis device 10 or module (the input module 20, the pre-processing module 30, and the post-processing module 60) may be further provided with a module buffer area, the track 40 may also be provided with a track buffer area, and the entire track 40 may be a cyclic track. It should be noted that each type of module shown in the figure (including the input module 20, the pre-processing module 30, and the post-processing module 60) is depicted as a single unit; however, it will be understood by those skilled in the art that the number of each type of module is not limited herein. For example, there may be multiple centrifugation modules 31, and multiple decapping modules 33, among others.

In some embodiments, the sample analysis system 100 may further include a processor 90.

In some embodiments, the processor 90 is configured to control one or more of the dispatching apparatus 50, the input module 20, the pre-processing module 30, and the post-processing module 60 to perform operations.

In some embodiments, the processor 90 is configured to execute each computer application program in a non-transitory computer-readable storage medium so as to perform corresponding steps and methods. For example, the processor 90 may be implemented by software, hardware, firmware, or a combination thereof, and may use at least one of a circuit, one or more application specific integrated circuits (ASIC), a digital signal processor (DSP), a digital signal processing device (DSPD), a programmable logic device (PLD), a field programmable gate array (FPGA), a central processing unit (CPU), a controller, a microcontroller, and a microprocessor, such that the processor 90 may perform some or all of the steps or any combination of the steps in the automatic review method in the embodiments of the disclosure, which will be described in detail below.

The foregoing is some descriptions of the sample analysis system 100.

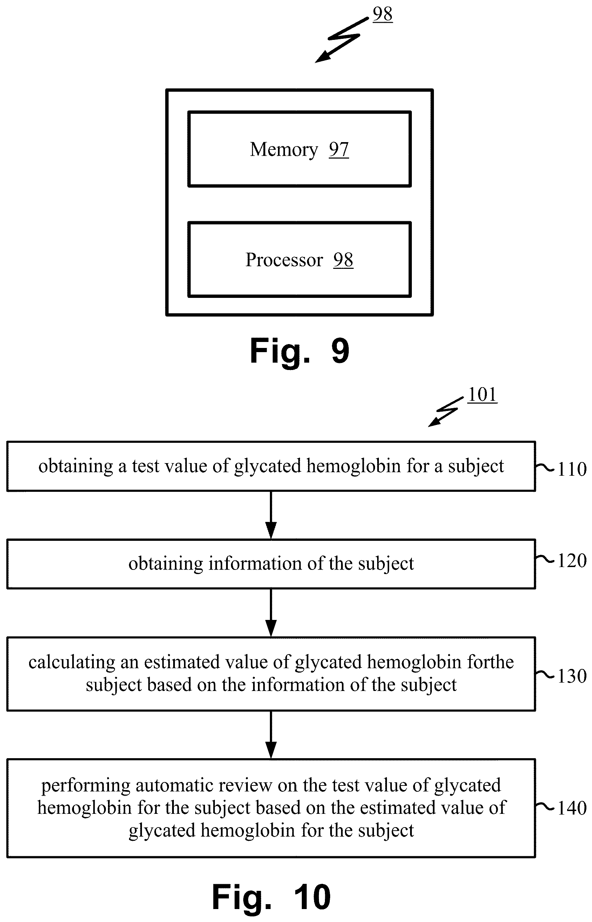

Referring to FIG. 9, an automatic review device 99 for glycated hemoglobin is disclosed in some embodiments. The automatic review device includes a memory 97 and a processor 98, which will be described in detail below. In some embodiments, the memory 97 is configured to store programs and/or data. In some embodiments, the memory 97 may be a tangible and non-transitory computer-readable medium, such as may be a flash memory card, a solid-state memory, or a hard disk. In some embodiments, the processor 98 is configured to execute programs stored in the memory 97 to perform corresponding steps and methods. For example, the processor 98 may be implemented by software, hardware, firmware, or a combination thereof, and may use at least one of a circuit, one or more application specific integrated circuits (ASIC), a digital signal processor (DSP), a digital signal processing device (DSPD), a programmable logic device (PLD), a field programmable gate array (FPGA), a central processing unit (CPU), a controller, a microcontroller, and a microprocessor, such that the processor 98 may perform some or all of the steps or any combination of the steps in the automatic review method in the embodiments of the disclosure, which will be described in detail below.

In some embodiments, the automatic review device 99 for glycated hemoglobin may be a device such as a computer or a mobile phone.

Referring to FIG. 10, an automatic review method 101 for glycated hemoglobin (hereinafter referred to as automatic review method 101) is disclosed in some embodiments of the disclosure. The method includes the following steps.

Step 110: obtaining a test value for glycated hemoglobin of a subject.

The test value for glycated hemoglobin obtained in step 110 is a test value obtained based on in vitro diagnostic technology. For example, the test value of glycated hemoglobin for the subject can be obtained by using the sample analysis device 10 or the analysis device 10 to test a sample of the subject.

Step 120: obtaining information of the subject.

In some embodiments, the information of the subject involved in step 120 includes basic information for the subject and/or an in vitro diagnostic test value associated with glycated hemoglobin for the subject. In other words, the information involved in step 120 includes the basic information and/or the in vitro diagnostic test value associated with glycated hemoglobin. It may be understood that the in vitro diagnostic test value associated with glycated hemoglobin refers to a test value obtained based on the in vitro diagnostic technology. In addition, test values mentioned herein are all actual values or measured values obtained through testing based on the in vitro diagnostic technology.

In some embodiments, the basic information for the subject includes basic information of first type and/or basic information of second type. The basic information of first type is quantifiable information that represents personal characteristic of the subject. The quantifiable information means that the information is numeric information and is represented by numeric values. For example, the basic information of first type includes at least one of followings: age, height, weight, and body mass index. The basic information of second type is non-quantifiable information that represents personal characteristic of the subject. The non-quantifiable information means that the information is not numeric information and is not represented by numeric values. For example, the basic information of second type includes at least one of followings: history of diabetes, medication status, and sample source department. It may be understood that although non-quantifiable information is not numeric information and is not represented by numerical values, some numerical values may be artificially defined to represent the non-quantifiable information. For example, 1 and 0 are used to respectively represent having a history of diabetes and not having a history of diabetes, or different numerical values are used to represent different sample source departments. In this way, the basic information of second type can be defined in a program and used as input.

Therefore, in some examples, the basic information for the subject includes at least one of followings: age, height, weight, body mass index, history of diabetes, medication status, and sample source department.

In some embodiments, the in vitro diagnostic test value associated with glycated hemoglobin includes at least one of followings: a test value related to blood glucose and a test value related to red blood cell. The detailed description is given below.

In some embodiments, the test value related to blood glucose includes: a test value that reflects a related substance involved in blood glucose metabolism and/or a test value that reflects a substance related to blood glucose concentration. In some examples, the test value that reflects the related substance involved in blood glucose metabolism includes at least one of followings: a related test value that reflects insulin, and a related test value that reflects a metabolite of insulin. In some examples, the related test value that reflects insulin includes at least one of followings: a test value of glutamate decarboxylase antibody, a test value of insulin autoantibody, a test value of islet cell antibody, a test value of islet cell antigen 2 antibody, and a test value of zinc transporter 8 antibody. In some examples, the related test value that reflects the metabolite of insulin includes at least one of followings: a test value of serum C-peptide, a test value of adiponectin, and a test value of fetuin. In some examples, the test value that reflects the substance related to blood glucose concentration includes at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin. In some examples, the test value of blood glucose includes at least one of followings: a test value of fasting blood glucose, a test value of 1-hour postprandial blood glucose, a test value of 2-hour postprandial blood glucose, and a test value of 3-hour postprandial blood glucose. In some examples, the test value of bilirubin includes at least one of followings: a test value of total bilirubin, a test value of direct bilirubin, and a test value of indirect bilirubin.

In some embodiments, the test value related to red blood cell includes at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.

In some embodiments, the in vitro diagnostic test value associated with glycated hemoglobin includes at least one of followings: a biochemical test value associated with glycated hemoglobin, a blood routine test value associated with glycated hemoglobin, an immunological test value associated with glycated hemoglobin, and a mass spectrometry test value associated with glycated hemoglobin. It may be understood that the biochemical test value associated with glycated hemoglobin is obtained through testing by a biochemical analysis device, the blood routine test value associated with glycated hemoglobin is obtained through testing by a blood analysis device, the immunological test value associated with glycated hemoglobin is obtained through testing by an immunological analysis device, and the mass spectrometry test value associated with glycated hemoglobin is obtained through testing by a mass spectrometry analysis device.

In some embodiments, the biochemical test value associated with glycated hemoglobin includes at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, and a test value of bilirubin. In some examples, the test value of blood glucose includes at least one of followings: a test value of fasting blood glucose, a test value of 1-hour postprandial blood glucose, a test value of 2-hour postprandial blood glucose, and a test value of 3-hour postprandial blood glucose. In some examples, the test value of bilirubin includes at least one of followings: a test value of total bilirubin, a test value of direct bilirubin, and a test value of indirect bilirubin.

In some embodiments, the blood routine test value associated with glycated hemoglobin includes at least one of followings: a test value of lactic acid, and a test value related to red blood cell. In some examples, the test value related to red blood cell includes at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.

In some embodiments, the immunological test value associated with glycated hemoglobin includes at least one of followings: a test value of glutamate decarboxylase antibody, a test value of insulin autoantibody, a test value of islet cell antibody, a test value of islet cell antigen 2 antibody, a test value of zinc transporter 8 antibody, a test value of serum C-peptide, a test value of adiponectin, and a test value of fetuin.

In some embodiments, the mass spectrometry test value associated with glycated hemoglobin includes at least one of followings: a test value of 1-anhydroglucitol, and a test value of 5-anhydroglucitol.

It may be understood that if the above-mentioned steps are performed by the processor 19 of the sample analysis device 10 to obtain the test value of glycated hemoglobin for the subject and the in vitro diagnostic test value associated with glycated hemoglobin, the sample analysis device 10 itself may test a sample of the subject to obtain the test value of glycated hemoglobin for the subject, and the in vitro diagnostic test value associated with glycated hemoglobin may be obtained by testing a sample of the subject using the sample analysis device 10 itself, or may be alternatively obtained by the sample analysis device 10 from another sample analysis device 10, which depends on the device type of the sample analysis device 10 itself and the type of test item that can be realized by the sample analysis device itself. Further, if the in vitro diagnostic test value associated with glycated hemoglobin includes at least two of the above-mentioned test values, some of the test values may be obtained by testing a sample of the subject using the sample analysis device 10 itself, and the remaining test values are obtained by the sample analysis device 10 from another sample analysis device 10, which also depends on the device type of the sample analysis device 10 itself and the type of test item that can be realized by the sample analysis device itself.

Similarly, if the above-mentioned steps are performed by the processor 90 of the sample analysis system 100 to obtain the test value of glycated hemoglobin for the subject and the in vitro diagnostic test value associated with glycated hemoglobin, one of the sample analysis devices 10 in the sample analysis system 100 may be used to test a sample of the subject to obtain the test value of glycated hemoglobin for the subject, and the in vitro diagnostic test value associated with glycated hemoglobin may be obtained by testing a sample of the subject using one or more of the sample analysis devices 10 included in the sample analysis system 100, or alternatively may be partially obtained by testing a sample of the subject using one more of the sample analysis devices 10 included in the sample analysis system 100 with the remaining portion obtained using another sample analysis device 10 that does not belong to the sample analysis system 100. This depends on the device types of the sample analysis devices 10 included in the sample analysis system 100 and the types of test items that can be realized by the sample analysis devices.

Step 130: calculating an estimated value of glycated hemoglobin for the subject based on the information of the subject. The detailed description is given below.

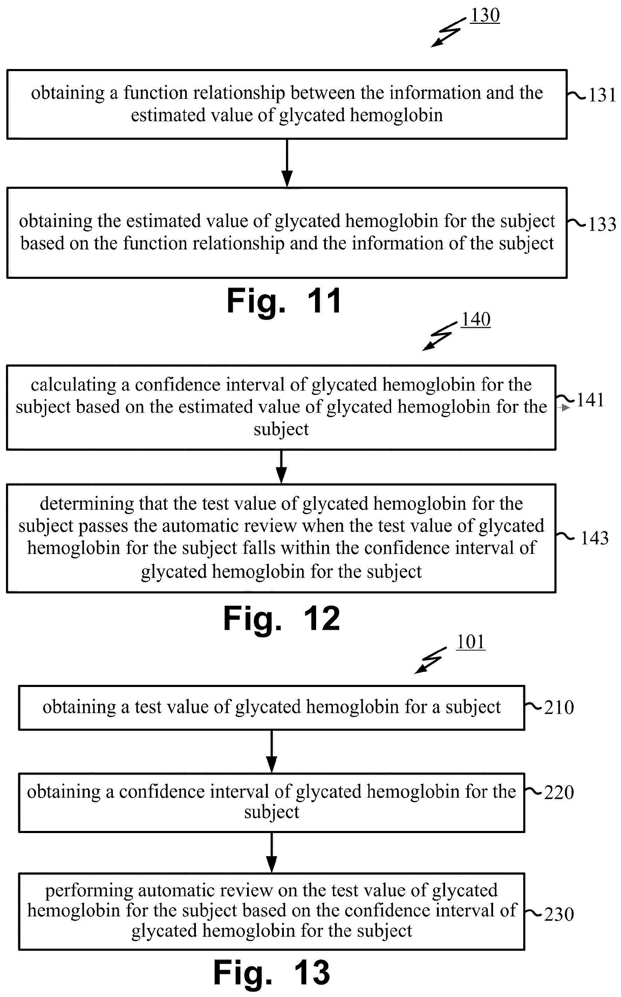

Referring to FIG. 11, in some embodiments, step 130 includes step 131 and step 133. In step 131, a function relationship between the information and the estimated value of glycated hemoglobin is obtained. In step 133, the estimated value of glycated hemoglobin for the subject is obtained based on the function relationship and the information of the subject. In some examples, the function relationship includes a first function formula. Accordingly, step 133 of obtaining the estimated value of glycated hemoglobin for the subject based on the function relationship and the information of the subject includes: inputting the information of the subject as an input value into the first function formula, so as to obtain the estimated value of glycated hemoglobin for the subject through calculation.

For example, in some examples where the information of the subject includes the basic information and the in vitro diagnostic test value associated with glycated hemoglobin, step 131 includes obtaining a function relationship between the basic information, the in vitro diagnostic test value associated with glycated hemoglobin, and the estimated value of glycated hemoglobin, and step 133 includes obtaining the estimated value of glycated hemoglobin for the subject based on the function relationship, the basic information for the subject and the in vitro diagnostic test value associated with glycated hemoglobin for the subject. For example, step 133 includes inputting the basic information for the subject and the in vitro diagnostic test value associated with glycated hemoglobin for the subject as input values into the first function formula, so as to obtain the estimated value of glycated hemoglobin for the subject through calculation.

For another example, in some examples where the information of the subject includes the basic information of first type and the in vitro diagnostic test value associated with glycated hemoglobin, step 131 includes obtaining a function relationship between the basic information of first type, the in vitro diagnostic test value associated with glycated hemoglobin, and the estimated value of glycated hemoglobin, and step 133 includes obtaining the estimated value of glycated hemoglobin for the subject based on the function relationship, the basic information of first type for the subject and the in vitro diagnostic test value associated with glycated hemoglobin for the subject. For example, step 133 includes inputting the basic information of first type for the subject and the in vitro diagnostic test value associated with glycated hemoglobin for the subject as input values into the first function formula, so as to obtain the estimated value of glycated hemoglobin for the subject through calculation.

For another example, in some examples where the information of the subject includes the basic information, the test value related to blood glucose and the test value related to red blood cell, step 131 includes obtaining a function relationship between the basic information, the test value related to blood glucose, the test value related to red blood cell, and the estimated value of glycated hemoglobin, and step 133 includes obtaining the estimated value of glycated hemoglobin for the subject based on the function relationship, the basic information for the subject, the test value related to blood glucose for the subject, the test value related to red blood cell for the subject and the estimated value of glycated hemoglobin for the subject. For example, step 133 includes inputting the basic information for the subject, the test value related to blood glucose for the subject and the test value related to red blood cell for the subject as input values into the first function formula, so as to obtain the estimated value of glycated hemoglobin for the subject through calculation.

For another example, in some examples where the information of the subject includes the basic information of first type, the test value related to blood glucose and the test value related to red blood cell, step 131 includes obtaining a function relationship between the basic information of first type, the test value related to blood glucose, the test value related to red blood cell, and the estimated value of glycated hemoglobin, and step 133 includes obtaining the estimated value of glycated hemoglobin for the subject based on the function relationship, the basic information of first type for the subject, the test value related to blood glucose for the subject and the test value related to red blood cell for the subject. For example, step 133 includes inputting the basic information of first type for the subject, the test value related to blood glucose for the subject and the test value related to red blood cell for the subject as input values into the first function formula, so as to obtain the estimated value of glycated hemoglobin for the subject through calculation.

It may be understood that the function relationship may be preset or generated in real time. Taking the sample analysis device 10 as an example, the function relationship may be preset in the sample analysis device 10 and is invoked when needed. The function relationship may alternatively be obtained and generated in real time when needed by the sample analysis device 10 through manners such as fitting. Similarly, the first function formula may also be preset or generated in real time, which are not described herein again.

The first function formula is explained below.

In some embodiments, the basic information of first type is age, the test value related to blood glucose is a test value of blood glucose, and the test value related to red blood cell is a test value of red blood cell count. The first function formula is: HbA1c' = a1 + a2*Age + a3*GLU + a4*RBC, where HbA1c' represents the estimated value of glycated hemoglobin, Age represents the age, GLU represents the test value of blood glucose, RBC represents the test value of red blood cell count, and a1, a2, a3, and a4 represent constants. In some examples, a1 ranges from 1.6 to 2.6, a2 ranges from 0.01 to 0.02, a3 ranges from 0.4 to 0.5, and a4 ranges from 0.07 to 0.24.

In some embodiments, the basic information of first type is age, the test value related to blood glucose includes a test value of fasting blood glucose, a test value of 2-hour postprandial blood glucose and a test value of bilirubin, and the test value related to red blood cell is a test value of red blood cell count. The first function formula is: HbA1c' = b1 + b2*Age + b3*GLU 1 + b4*G120 + b5/IB² + b6*RBC; where HbA1c' represents the estimated value of glycated hemoglobin, Age represents age, GLU1 represents fasting blood glucose, G120 represents 2-hour postprandial blood glucose, IB represents the test value of bilirubin, RBC represents the test value of red blood cell count, and b1, b2, b3, b4, b5, and b6 represent constants. In some examples, b1 ranges from 1.6 to 2.5, b2 ranges from 0.001 to 0.009, b3 ranges from 0.24 to 0.3, b4 ranges from 0.09 to 0.13, b5 ranges from 2.5 to 10.9, and b6 ranges from 0.13 to 0.29.

In some embodiments, the basic information of first type is age, the test value related to blood glucose includes a test value of blood glucose and a test value of bilirubin, and the test value related to red blood cell is a test value of red blood cell count. The first function formula is: HbA1c' = c1 + c2*Age + c3*GLU + c4/IB² + c5*RBC; where HbA1c' represents the estimated value of glycated hemoglobin, Age represents age, GLU represents the test value of blood glucose, IB represents the test value of bilirubin, RBC represents the test value of red blood cell count, and c1, c2, c3, c4, and c5 represent constants. In some examples, c1 ranges from 1.2 to 2.3, c2 ranges from 0.01 to 0.02, c3 ranges from 0.4 to 0.47, c4 ranges from 2.8 to 12.97, and c5 ranges from 0.1 to 0.29.

In some embodiments, step 130 may be implemented by machine learning. For example, step 130 includes inputting the information of the subject into a glycated hemoglobin estimation model, so as to obtain the estimated value of glycated hemoglobin for the subject through calculation. In some examples, the glycated hemoglobin estimation model is configured to use the information of the subject as input data, and perform calculation to output the estimated value of glycated hemoglobin for the subject. In some examples, the glycated hemoglobin estimation model is obtained through training by using a training set. Data of the training set includes first data and second data, where the first data is labeled with the second data, the first data includes information of other subjects, and the second data includes test values of glycated hemoglobin for said subjects.

For example, in some examples where the information of the subject includes the basic information and the in vitro diagnostic test value associated with glycated hemoglobin, step 130 includes inputting the basic information for the subject and the in vitro diagnostic test value associated with glycated hemoglobin for the subject into the glycated hemoglobin estimation model, so as to obtain the estimated value of glycated hemoglobin for the subject through calculation. The glycated hemoglobin estimation model is configured to use the basic information and the in vitro diagnostic test value associated with glycated hemoglobin as input data, and perform calculation to output the estimated value of glycated hemoglobin for the subject. The glycated hemoglobin estimation model is obtained through training by using a training set. Data of the training set includes first data and second data, where the first data is labeled with the second data, the first data includes basic information and in vitro diagnostic test values associated with glycated hemoglobin for other subjects, and the second data includes test values of glycated hemoglobin for said subjects.

For another example, in some examples where the information of the subject includes the basic information of first type and the in vitro diagnostic test value associated with glycated hemoglobin, step 130 includes inputting the basic information of first type for the subject and the in vitro diagnostic test value associated with glycated hemoglobin for the subject into the glycated hemoglobin estimation model, so as to obtain the estimated value of glycated hemoglobin for the subject through calculation. The glycated hemoglobin estimation model is configured to use the basic information of first type and the in vitro diagnostic test value associated with glycated hemoglobin as input data, and perform calculation to output the estimated value of glycated hemoglobin for the subject. The glycated hemoglobin estimation model is obtained through training by using a training set. Data of the training set includes first data and second data, where the first data is labeled with the second data, the first data includes first-type basic information and in vitro diagnostic test values associated with glycated hemoglobin for other subjects, and the second data includes test values of glycated hemoglobin for said subjects.

For another example, in some examples where the information of the subject includes the basic information, the test value related to blood glucose and the test value related to red blood cell, step 130 includes inputting the basic information for the subject, the test value related to blood glucose of the subject and the test value related to red blood cell into the glycated hemoglobin estimation model, so as to obtain the estimated value of glycated hemoglobin for the subject through calculation. The glycated hemoglobin estimation model is configured to use the basic information, the test value related to blood glucose and the test value related to red blood cell as input data, and perform calculation to output the estimated value of glycated hemoglobin for the subject. The glycated hemoglobin estimation model is obtained through training by using a training set. Data of the training set includes first data and second data, where the first data is labeled with the second data, the first data includes basic information, test values related to blood glucose and test values related to red blood cell for other subjects, and the second data includes test values of glycated hemoglobin for said subjects.

For another example, in some examples where the information of the subject includes the basic information of first type, the test value related to blood glucose and the test value related to red blood cell, step 130 includes inputting the basic information of first type for the subject, the test value related to blood glucose for the subject and the test value related to red blood cell for the subject into the glycated hemoglobin estimation model, so as to obtain the estimated value of glycated hemoglobin for the subject through calculation. The glycated hemoglobin estimation model is configured to use the basic information of first type, the test value related to blood glucose and the test value related to red blood cell as input data, and perform calculation to output the estimated value of glycated hemoglobin for the subject. The glycated hemoglobin estimation model is obtained through training by using a training set. Data of the training set includes first data and second data, where the first data is labeled with the second data, the first data includes first-type basic information, test values related to blood glucose and test values related to red blood cell for other subjects, and the second data includes test values of glycated hemoglobin for said subjects.

In some embodiments, the glycated hemoglobin estimation model includes: a convolutional neural network-based model, a recurrent neural network-based model, a generative adversarial neural network-based model, an attention neural network-based model, or a fully connected network-based model.

Step 140: performing an automatic review on the test value of glycated hemoglobin for the subject based on the estimated value of glycated hemoglobin for the subject. In some embodiments, step 140 includes calculating a matching degree between the estimated value and the test value of glycated hemoglobin for the subject, and performing the automatic review on the test value of glycated hemoglobin for the subject based on the matching degree. With step 140 of performing the automatic review on the test value of glycated hemoglobin for the subject, it may be determined whether said test value is accurate.

Referring to FIG. 12, in some embodiments, step 140 of performing automatic review includes the following steps.

Step 141: calculating a confidence interval of glycated hemoglobin for the subject based on the estimated value of glycated hemoglobin for the subject. In some examples, step 141 includes calculating the confidence interval of glycated hemoglobin for the subject based on the estimated value of glycated hemoglobin for the subject and a second function formula. In some examples, the second function formula includes: CI1 = HbA1c' - D1; CI2 = HbA1c' + D2, where HbA1c' represents the estimated value of glycated hemoglobin, and D1 and D2 represent constants. The confidence interval CI for glycated hemoglobin of the subject satisfies: CI = [CI1, CI2], where CI1 represents a left endpoint of the confidence interval, CI2 represents a right endpoint of the confidence interval, and [CI1, CI2] represents an interval constituted by the two endpoints. In some examples, D1 is determined based on a standard deviation of glycated hemoglobin of a sample library and a preset confidence level. In some examples, D2 is determined based on a standard deviation of glycated hemoglobin of a sample library and a preset confidence level. In some examples, the sample library includes test values of glycated hemoglobin for a plurality of samples, i.e., glycated hemoglobin tests for a plurality of subjects. It may be understood that these test values are generally accurate and reliable. In some examples, D1 and D2 are equal.

In some examples, step 141 includes calculating an 80% or higher confidence interval of glycated hemoglobin for the subject based on the estimated value of glycated hemoglobin for the subject. In some examples, step 141 includes calculating an 83% or higher confidence interval of glycated hemoglobin for the subject based on the estimated value of glycated hemoglobin for the subject. In some examples, step 141 includes calculating an 85% or higher confidence interval of glycated hemoglobin for the subject based on the estimated value of glycated hemoglobin for the subject. In some examples, step 141 includes calculating an 87% or higher confidence interval of glycated hemoglobin for the subject based on the estimated value of glycated hemoglobin for the subject. In some examples, step 141 includes calculating a 90% or higher confidence interval of glycated hemoglobin for the subject based on the estimated value of glycated hemoglobin for the subject. In some examples, step 141 includes calculating a 92% or higher confidence interval of glycated hemoglobin for the subject based on the estimated value of glycated hemoglobin for the subject. In some examples, step 141 includes calculating a 93% or higher confidence interval of glycated hemoglobin for the subject based on the estimated value of glycated hemoglobin for the subject. In some examples, step 141 includes calculating a 95% or higher confidence interval of glycated hemoglobin for the subject based on the estimated value of glycated hemoglobin for the subject. In some examples, step 141 includes calculating a 97% or higher confidence interval of glycated hemoglobin for the subject based on the estimated value of glycated hemoglobin for the subject.

Step 143: determining that the test value of glycated hemoglobin for the subject passes the automatic review, that is, determining that the test value of glycated hemoglobin for the subject is accurate, when the test value of glycated hemoglobin for the subject falls within the confidence interval of glycated hemoglobin for the subject.

Generally, in the field of statistics, after a confidence interval for a parameter (referred to as parameter x) is obtained through calculation, it is applicable to each individual Object. For example, it is determined whether the value of parameter x for object 1 falls within the confidence interval, and if it does, it indicates a higher level of accuracy and a greater reliability, otherwise it indicates lower accuracy; it is determined whether the value of parameter x for object 2 falls within the confidence interval, and if it does, it indicates a higher level of accuracy and a greater reliability, otherwise it indicates lower accuracy; it is determined whether the value of parameter x for object 3 falls within the confidence interval, and if it does, it indicates a higher level of accuracy and a greater reliability, otherwise it indicates lower accuracy. That is, the same confidence interval is applicable to different objects. However, in some examples of the disclosure, the test values of glycated hemoglobin of different subjects are not adapted to be evaluated by a same confidence interval of glycated hemoglobin, but each subject has his own confidence interval of glycated hemoglobin. This is usually manifested as different subjects having different confidence intervals for glycated hemoglobin. For example, subject 1 has confidence interval 1 for glycated hemoglobin of subject 1, subject 2 has confidence interval 2 for glycated hemoglobin of subject 1, subject 3 has confidence interval 3 for glycated hemoglobin of subject 3, etc. The confidence interval 1, the confidence interval 2, and the confidence interval 3 are numerically different. The test value of glycated hemoglobin for the subject 1 is automatically reviewed by using the confidence interval 1 for glycated hemoglobin of the subject 1, the test value of glycated hemoglobin for the subject 2 is automatically reviewed by using the confidence interval 2 for glycated hemoglobin of the subject 2, and the test value of glycated hemoglobin for the subject 3 is automatically reviewed by using the confidence interval 3 for glycated hemoglobin of the subject 3. The test value for glycated hemoglobin of each subject is automatically reviewed by using its confidence interval of glycated hemoglobin. Results from this automatic review manner are more reliable and trustworthy.

As an example, the first function formula is: HbA1c' = a1 + a2*Age + a3*GLU + a4*RBC, after assigning values to a1, a2, a3, and a4 within their respective value ranges, the applicant collected test values of glycated hemoglobin of 1494 subjects to verify the automatic review method 101 of the disclosure. The test values of glycated hemoglobin of the 1494 subjects are test values that have already passed review and are reportable. Specifically, when the automatic review method 101 of the disclosure is used to perform automatic review on the test values of glycated hemoglobin of the 1494 subjects, for any subject among the 1494 subjects, the estimated value of glycated hemoglobin of said subject is obtained through calculation using the aforementioned first function formula, and the confidence interval of glycated hemoglobin of said subject is further calculated; if the test value for glycated hemoglobin of said subject falls within the confidence interval of glycated hemoglobin of said subject, it means that the automatic review method 101 of the disclosure determines that the test value for glycated hemoglobin of said subject passes the automatic review, and the test value for glycated hemoglobin of said subject is accurate. As verified: at a 95% confidence interval, among the 1494 subjects, 1426 subjects are determined to pass the review by the automatic review method 101 of the disclosure, and 68 subjects are determined to fail the review by the automatic review method 101 of the disclosure, with an accuracy rate of 95.23%; at a 92% confidence interval, among the 1494 subjects, 1378 subjects are determined to pass the review by the automatic review method 101 of the disclosure, and 116 subjects are determined to fail the review by the automatic review method 101 of the disclosure, with an accuracy rate of 92.24%; and at a 84% confidence interval, among the 1494 subjects, 1248 subjects are determined to pass the review by the automatic review method 101 of the disclosure, and 246 subjects are determined to fail the review by the automatic review method 101 of the disclosure, with an accuracy rate of 83.53%. Therefore, the validation shows that the automatic review method 101 of the disclosure exhibits good performance, thereby reducing workload of a reviewer, allowing the reviewer to focus on reviewing a small number of glycated hemoglobin test values that do not pass the automatic review and are suspected to be problematic. This contributes to improving efficiency of reviewing test reports and improving quality of test reports.

Still as an example, the first function formula is: HbA1c' = a1 + a2*Age + a3*GLU + a4*RBC, after a1, a2, a3, and a4 are assigned values within their respective value ranges, the values assigned this time are different from the values assigned used for the 1494 subjects described above. In addition, test values of glycated hemoglobin of another 1000 subjects were collected this time to verify the automatic review method 101 of the disclosure. Similarly, the test values of glycated hemoglobin of the 1000 subjects are test values that have pass the review and are reportable. As verified: at a 95% confidence interval, among the 1000 subjects, 952 subjects are determined to pass the review by the automatic review method 101 of the disclosure, and 48 subjects are determined to fail the review by the automatic review method 101 of the disclosure, with an accuracy rate of 95.2%; at a 93% confidence interval, among the 1000 subjects, 925 subjects are determined to pass the review by the automatic review method 101 of the disclosure, and 75 subjects are determined to fail the review by the automatic review method 101 of the disclosure, with an accuracy rate of 92.5%; and at a 83% confidence interval, among the 1000 subjects, 826 subjects are determined to pass the review by the automatic review method 101 of the disclosure, and 174 subjects are determined to fail the review by the automatic review method 101 of the disclosure, with an accuracy rate of 82.6%. Therefore, the validation shows that the automatic review method 101 of the disclosure exhibits good performance, thereby reducing workload of a reviewer, allowing the reviewer to focus on reviewing a small number of glycated hemoglobin test values that do not pass the automatic review and are suspected to be problematic. This contributes to improving efficiency of reviewing test reports and improving quality of test reports.

As an example, the first function formula is: HbA1c' = b1 + b2*Age + b3*GLU 1 + b4*G120 + b5/IB² + b6*RBC, after assigning values to b1, b2, b3, b4, b5, and b6 within their respective value ranges, the applicant collected test values of glycated hemoglobin of 814 subjects to verify the automatic review method 101 of the disclosure. Similarly, the test values of glycated hemoglobin of the 814 subjects are test values that have pass the review and are reportable. As verified: at a 97% confidence interval, among the 814 subjects, 794 subjects are determined to pass the review by the automatic review method 101 of the disclosure, and 20 subjects are determined to fail the review by the automatic review method 101 of the disclosure, with an accuracy rate of 97.54% ; and at a 94% confidence interval, among the 814 subjects, 765 subjects are determined to pass the review by the automatic review method 101 of the disclosure, and 49 subjects are determined to fail the review by the automatic review method 101 of the disclosure, with an accuracy rate of 93.98%. Therefore, the validation shows that the automatic review method 101 of the disclosure exhibits good performance, thereby reducing workload of a reviewer, allowing the reviewer to focus on reviewing a small number of glycated hemoglobin test values that do not pass the automatic review and are suspected to be problematic. This contributes to improving efficiency of reviewing test reports and improving quality of test reports.

As an example, the first function formula is: HbA1c' = c1 + c2*Age + c3*GLU + c4/IB² + c5*RBC, after assigning values to c1, c2, c3, c4, and c5 within their respective value ranges, the applicant collected test values of glycated hemoglobin of 2000 subjects to verify the automatic review method 101 of the disclosure. Similarly, the test values of glycated hemoglobin of the 2000 subjects are test values that have pass the review and are reportable. As verified: at a 97% confidence interval, among the 2000 subjects, 1939 subjects are determined to pass the review by the automatic review method 101 of the disclosure, and 61 subjects are determined to fail the review by the automatic review method 101 of the disclosure, with an accuracy rate of 96.95%; at a 93.6% confidence interval, among the 2000 subjects, 1871 subjects are determined to pass the review by the automatic review method 101 of the disclosure, and 129 subjects are determined to fail the review by the automatic review method 101 of the disclosure, with an accuracy rate of 93.55%; and at a 80.1% confidence interval, among the 2000 subjects, 1602 subjects are determined to pass the review by the automatic review method 101 of the disclosure, and 398 subjects are determined to fail the review by the automatic review method 101 of the disclosure, with an accuracy rate of 80.1%. Therefore, the validation shows that the automatic review method 101 of the disclosure exhibits good performance, thereby reducing workload of a reviewer, allowing the reviewer to focus on reviewing a small number of glycated hemoglobin test values that do not pass the automatic review and are suspected to be problematic. This contributes to improving efficiency of reviewing test reports and improving quality of test reports.

In some embodiments, the automatic review in step 140 includes: outputting the test value of glycated hemoglobin for the subject when the test value of glycated hemoglobin for the subject and the estimated value of glycated hemoglobin for the subject satisfy a preset relationship; or prompting a manual review when the test value of glycated hemoglobin for the subject and the estimated value of glycated hemoglobin for the subject do not satisfy the preset relationship.

Whether the preset relationship is satisfied may be whether a matching degree between the estimated value and the test value of glycated hemoglobin for the subject is high, for example, whether the matching degree between the estimated value and the test value of glycated hemoglobin for the subject is greater than a matching degree threshold, and if the matching degree is greater than the matching degree threshold, the matching degree between the estimated value and the test value is high, otherwise, the preset relationship is not satisfied.

Whether the preset relationship is satisfied may be whether the test value of glycated hemoglobin for the subject falls within the confidence interval of glycated hemoglobin of the subject that is calculated based on the estimated value of glycated hemoglobin for the subject, and if the test value falls within the confidence interval, the preset relationship is satisfied, otherwise, the preset relationship is not satisfied.

In some embodiments, the automatic review method 101 further includes the following steps: prompting a manual review when the information of the subject cannot be obtained.

For example, in some examples where the information of the subject includes the basic information and the in vitro diagnostic test value associated with glycated hemoglobin, if the basic information for the subject cannot be obtained or the in vitro diagnostic test value associated with glycated hemoglobin cannot be obtained, a manual review is prompted.

For another example, in some examples where the information of the subject includes the basic information of first type and the in vitro diagnostic test value associated with glycated hemoglobin, if the basic information of first type for the subject cannot be obtained or the in vitro diagnostic test value associated with glycated hemoglobin cannot be obtained, a manual review is prompted.

For another example, in some examples where the information of the subject includes the basic information, the test value related to blood glucose, and the test value related to red blood cell, if the basic information for the subject, the test value related to blood glucose, or the test value related to red blood cell cannot be obtained, a manual review is prompted.

For another example, in some examples where the information of the subject includes the basic information of first type, the test value related to blood glucose, and the test value related to red blood cell, if the basic information of first type for the subject, the test value related to blood glucose, or the test value related to red blood cell cannot be obtained, a manual review is prompted. For example, if the first function formula is: HbA1c' = a1 + a2*Age + a3*GLU + a4*RBC, manual review is prompted when the age of the subject, a test value of blood glucose of the subject, or a test value of red blood cell count of the subject cannot be obtained. For another example, if the first function formula is: HbA1c' = b1 + b2*Age + b3*GLU 1 + b4*G120 + b5/IE² + b6*RBC, manual review is prompted when the age of the subject, a test value of fasting blood glucose of the subject, a test value of 2-hour postprandial blood glucose of the subject, a test value of bilirubin of the subject, or a test value of red blood cell count of the subject is not cannot be obtained. For another example, if the first function formula is: HbA1c' = c1 + c2*Age + c3*GLU + c4/IE² + c5*RBC, manual review is prompted when the age of the subject, a test value of fasting blood glucose of the subject, a test value of bilirubin of the subject, or a test value of red blood cell count of the subject cannot be obtained.

Referring to FIG. 13, an automatic review method 101 for glycated hemoglobin in some embodiments of the disclosure includes the following steps:
Step 210: obtaining a test value for glycated hemoglobin of a subject.

The test value for glycated hemoglobin obtained in step 210 is a test value obtained based on in vitro diagnostic technology. For example, the test value of glycated hemoglobin for the subject can be obtained by using the sample analysis device 10 or the analysis device 10 to test a sample of the subject.

step 220: obtaining a confidence interval of glycated hemoglobin for the subject, where the confidence interval of glycated hemoglobin for the subject is configured to evaluate the test value of glycated hemoglobin for the subject.

In some embodiments, the confidence interval of glycated hemoglobin of the subject is obtained through calculation based on the estimated value of glycated hemoglobin for the subject. Specifically, for the manner of calculating the confidence interval of glycated hemoglobin of the subject based on the estimated value of glycated hemoglobin for the subject, reference may be made to the description of step 141 above. For example, the confidence interval of glycated hemoglobin for the subject is obtained through calculation based on the estimated value of glycated hemoglobin for the subject and the second function formula. In some examples, the second function formula includes: CI1 = HbA1c' - D1; CI2 = HbA1c' + D2, where HbA1c' represents the estimated value of glycated hemoglobin, and D1 and D2 represent constants. The confidence interval CI for glycated hemoglobin of the subject satisfies: CI = [CI1, CI2], where CI1 represents a left endpoint of the confidence interval, CI2 represents a right endpoint of the confidence interval, and [CI1, CI2] represents an interval constituted by the two endpoints. In some examples, D1 is determined based on a standard deviation of glycated hemoglobin of a sample library and a preset confidence level. In some examples, D2 is determined based on a standard deviation of glycated hemoglobin of a sample library and a preset confidence level. In some examples, the sample library includes test values of glycated hemoglobin of a plurality of samples. In some examples, D1 and D2 are equal.

In some embodiments, the estimated value of glycated hemoglobin for the subject is obtained through calculation based on the information of the subject. Specifically, for the manner obtaining the estimated value of glycated hemoglobin for the subject through calculation based on the information of the subject, reference may be made to the description of step 130 above. For example, a function relationship between the information of the subject and the estimated value of glycated hemoglobin is obtained, and the estimated value of glycated hemoglobin for the subject is obtained based on the function relationship and the information of the subject. In some examples, the function relationship includes a first function formula. For the first function formula, reference may be made to the description above and will not be described herein again.

In some embodiments, the confidence interval of glycated hemoglobin for the subject is obtained through calculation based on a mean and a standard deviation of the test values of glycated hemoglobin of the plurality of samples in the sample library. For example, the confidence interval CI for glycated hemoglobin of the subject satisfies: CI = AV ± d*Std, where AV represents the mean of the test values of glycated hemoglobin of the plurality of samples in the sample library, Std represents the standard deviation of the test values of glycated hemoglobin for the plurality of samples in the sample library, and d is a constant whose value may be assigned based on requirements. Generally, d is determined by a preset confidence level. In some examples, the sample library includes test values of glycated hemoglobin of a plurality of samples, i.e., test values of glycated hemoglobin of a plurality of subjects. It may be understood that these test values are generally accurate and reliable.

Step 230: performing an automatic review on the test value of glycated hemoglobin for the subject based on the confidence interval of glycated hemoglobin for the subject.

In some embodiments, the automatic review in step 230 includes: outputting the test value of glycated hemoglobin for the subject when the test value of glycated hemoglobin for the subject and the estimated value of glycated hemoglobin for the subject satisfy a preset relationship; or prompting a manual review when the test value of glycated hemoglobin for the subject and the estimated value of glycated hemoglobin for the subject do not satisfy the preset relationship.

Whether the preset relationship is satisfied may be whether the test value of glycated hemoglobin for the subject falls within the confidence interval of glycated hemoglobin for the subject, and if the test value falls within the confidence interval, the preset relationship is satisfied, otherwise, the preset relationship is not satisfied.

Descriptions are provided herein with reference to various exemplary embodiments. However, those skilled in the art should understand that variations and corrections may be made to the exemplary embodiments without departing from the scope of this specification. For example, various operational steps and assemblies for executing the operational steps may be implemented in different methods according to specific applications or in consideration of any number of cost functions associated with operations of the system (for example, one or more steps may be deleted, modified or incorporated into other steps).

In the foregoing embodiments, the disclosure may be implemented in whole or in part by software, hardware, firmware, or any combination thereof. In addition, those skilled in the art can understand that the principles herein may be reflected in a computer program product in a computer-readable storage medium, where the readable storage medium is loaded with computer-readable program codes in advance. Any tangible non-transitory computer-readable storage medium may be used, including a magnetic storage device (a hard disk, a floppy disk, or the like), an optical storage device (CD-ROM, DVD, Blu-ray disc, or the like), a flash memory, and/or the like. These computer program instructions may be loaded on a general-purpose computer, a special-purpose computer, or other programmable data processing devices to form a machine, such that these instructions, when executed on a computer or other programmable data processing apparatus, may produce a means for implementing a specified function. These computer program instructions may alternatively be stored in a computer-readable memory, which may instruct a computer or other programmable data processing device to operate in a particular manner, such that the instructions stored in the computer-readable memory may produce an article of manufacture which includes a means for implementing a specified function. The computer program instructions may alternatively be loaded onto a computer or other programmable data processing device to perform a series of operational steps on the computer or other programmable device to produce a computer-implemented process, such that the instructions, when executed on the computer or other programmable device, may provide steps for implementing a specified function.

Although the principles herein are shown in various embodiments, many modifications of structures, arrangements, proportions, elements, materials, and components particularly applicable to specific environmental and operating requirements may be made without departing from the principles and scope of the disclosure. These modifications and other changes or corrections fall within the scope of this specification.

The foregoing detailed descriptions are provided with reference to various embodiments. However, those skilled in the art should understand that various corrections and changes may be made without departing from the scope of the disclosure. Therefore, the disclosure is intended for an illustrative purpose other than a limitative purpose, and all these modifications fall within the scope of the disclosure. Similarly, the advantages, other advantages, and solutions to problems of the various embodiments are described above. However, the benefits, the advantages, the solutions to the problems, and any of their contributing factors, or solutions clarifying them should not be construed to be critical, necessary, or essential. The term "include" or "including" used herein and any other variations thereof all refer to a non-exclusive inclusion, such that a process, method, article, or device including a list of elements includes not only these elements, but also other elements that are not expressly listed or not inherent to the process, method, system, article, or device. In addition, the term "couple" or "coupling" used herein and any other variations thereof refer to a physical connection, an electrical connection, a magnetic connection, an optical connection, a communication connection, a functional connection, and/or any other connections.

Those skilled in the art should understand that many changes may be made to the details of the foregoing embodiments without departing from the basic principles of the disclosure. Therefore, the scope of the disclosure should be determined by only the claims.

### EXAMPLES

1. An automatic review method for glycated hemoglobin, characterized in that the method comprises:
   testing a sample of a subject to obtain a test value of glycated hemoglobin for the subject;
   obtaining basic information of first type for the subject, wherein the basic information of first type is quantifiable information that represents personal characteristic of the subject;
   obtaining a test value related to blood glucose for the subject and a test value related to red blood cell for the subject;
   calculating an estimated value of glycated hemoglobin for the subject based on the basic information of first type, the test value related to blood glucose and the test value related to red blood cell; and
   performing an automatic review on the test value of glycated hemoglobin for the subject based on the estimated value of glycated hemoglobin for the subject.
2. The automatic review method of example 1, characterized in that the test value related to blood glucose comprises: a test value that reflects a related substance involved in blood glucose metabolism and/or a test value that reflects a substance related to blood glucose concentration;
   preferably, the test value that reflects the related substance involved in blood glucose metabolism comprises at least one of followings: a related test value that reflects insulin, and a related test value that reflects a metabolite of insulin; preferably, the related test value that reflects insulin comprises at least one of followings: a test value of glutamate decarboxylase antibody, a test value of insulin autoantibody, a test value of islet cell antibody, a test value of islet cell antigen 2 antibody, and a test value of zinc transporter 8 antibody; the related test value that reflects the metabolite of insulin comprises at least one of followings: a test value of serum C-peptide, a test value of adiponectin, and a test value of fetuin;
   preferably, the test value that reflects the substance related to blood glucose concentration comprises at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin; preferably, the test value of blood glucose comprises at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin comprises at least one of followings: a test value of total bilirubin, a test value of direct bilirubin, and a test value of indirect bilirubin.
3. The automatic review method of example 1, characterized in that the test value related to red blood cell comprises at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.
4. The automatic review method of example 1, characterized in that the basic information of first type comprises at least one of followings: age, height, weight, and body mass index.
5. The automatic review method of any one of examples 1 to 4, characterized in that calculating an estimated value of glycated hemoglobin for the subject based on the basic information of first type, the test value related to blood glucose and the test value related to red blood cell, comprises:
   inputting the basic information of first type, the test value related to blood glucose and the test value related to red blood cell into a glycated hemoglobin estimation model, so as to obtain the estimated value of glycated hemoglobin for the subject through calculation, wherein the glycated hemoglobin estimation model is configured to use the basic information of first type, the test value related to blood glucose and the test value related to red blood cell as input data, and perform calculation to output the estimated value of glycated hemoglobin for the subject;
   wherein the glycated hemoglobin estimation model is obtained through training by using a training set, wherein data of the training set comprises first data and second data, the first data is labeled with the second data, the first data comprises first-type basic information, test values related to blood glucose and test values related to red blood cell for other subjects, and the second data comprises test values of glycated hemoglobin for said subjects.
6. The automatic review method of example 5, characterized in that the glycated hemoglobin estimation model comprises: a convolutional neural network-based model, a recurrent neural network-based model, a generative adversarial neural network-based model, an attention neural network-based model, or a fully connected network-based model.
7. The automatic review method of any one of examples 1 to 4, characterized in that calculating an estimated value of glycated hemoglobin for the subject based on the basic information of first type, the test value related to blood glucose and the test value related to red blood cell, comprises:
   obtaining a function relationship between the basic information of first type, the test value related to blood glucose, the test value related to red blood cell, and the estimated value of glycated hemoglobin; and
   obtaining the estimated value of glycated hemoglobin for the subject based on the function relationship, the basic information of first type for the subject, the test value related to blood glucose for the subject, and the test value related to red blood cell for the subject.
8. The automatic review method of example 7, characterized in that the function relationship comprises a first function formula; obtaining the estimated value of glycated hemoglobin for the subject based on the function relationship, the basic information of first type for the subject, the test value related to blood glucose for the subject, and the test value related to red blood cell for the subject, comprises:
   inputting the basic information of first type for the subject, the test value related to blood glucose for the subject, and the test value related to red blood cell for the subject as input values into the first function formula, so as to obtain the estimated value of glycated hemoglobin for the subject through calculation.
9. The automatic review method of example 8, characterized in that the first function formula comprises a function formula that preforms linear weighting on a plurality of input values.
10. The automatic review method of example 8 or 9, characterized in that the basic information of first type is age, the test value related to blood glucose is a test value of blood glucose, the test value related to red blood cell is a test value of red blood cell count, and the first function formula is: HbA1c' = a1 + a2*Age + a3*GLU + a4*RBC, wherein HbA1c' represents the estimated value of glycated hemoglobin, Age represents the age, GLU represents the test value of blood glucose, RBC represents the test value of red blood cell count, and a1, a2, a3, and a4 represent constants; preferably, a1 ranges from 1.6 to 2.6, a2 ranges from 0.01 to 0.02, a3 ranges from 0.4 to 0.5, and a4 ranges from 0.07 to 0.24; or
   the basic information of first type is age, the test value related to blood glucose comprises a test value of fasting blood glucose, a test value of 2-hour postprandial blood glucose and a test value of bilirubin, the test value related to red blood cell is a test value of red blood cell count, and the first function formula is: HbA1c' = b1 + b2*Age + b3*GLU 1 + b4*G120 + b5/IB² + b6*RBC, wherein HbA1c' represents the estimated value of glycated hemoglobin, Age represents the age, GLU1 represents the test value of fasting blood glucose, G120 represents the test value of 2-hour postprandial blood glucose, IB represents the test value of bilirubin, RBC represents the test value of red blood cell count, and b1, b2, b3, b4, b5, and b6 represent constants; preferably, b1 ranges from 1.6 to 2.5, b2 ranges from 0.001 to 0.009, b3 ranges from 0.24 to 0.3, b4 ranges from 0.09 to 0.13, b5 ranges from 2.5 to 10.9, and b6 ranges from 0.13 to 0.29; or
   the basic information of first type is age, the test value related to blood glucose comprises a test value of blood glucose and a test value of bilirubin, the test value related to red blood cell is a test value of red blood cell count, and the first function formula is: HbA1c' = c1 + c2*Age + c3*GLU + c4/IB² + c5*RBC, wherein HbA1c' represents the estimated value of glycated hemoglobin, Age represents the age, GLU represents the test value of blood glucose, IB represents the test value of bilirubin, and RBC represents the test value of red blood cell count, and c1, c2, c3, c4, and c5 represent constants; preferably, c1 ranges from 1.2 to 2.3, c2 ranges from 0.01 to 0.02, c3 ranges from 0.4 to 0.47, c4 ranges from 2.8 to 12.97, and c5 ranges from 0.1 to 0.29.
11. The automatic review method of example 1, characterized in that performing an automatic review on the test value of glycated hemoglobin for the subject based on the estimated value of glycated hemoglobin for the subject, comprises: calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate.
12. The automatic review method of example 11, characterized in that calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate, comprises:
   calculating a confidence interval of glycated hemoglobin for the subject based on the estimated value of glycated hemoglobin for the subject; and
   determining that the test value of glycated hemoglobin for the subject is accurate when the test value of glycated hemoglobin for the subject falls within the confidence interval of glycated hemoglobin for the subject.
13. The automatic review method of example 12, characterized in that calculating a confidence interval of glycated hemoglobin for the subject based on the estimated value of glycated hemoglobin for the subject, comprises: obtaining the confidence interval of glycated hemoglobin for the subject through calculation based on the estimated value of glycated hemoglobin for the subject and a second function formula.
14. The automatic review method of example 13, characterized in that the second function formula comprises: CI1 = HbA1c' - D1; CI2 = HbA1c' + D2, wherein HbA1c' represents the estimated value of glycated hemoglobin, and D1 and D2 represent constants;
   the confidence interval CI of glycated hemoglobin for the subject satisfies: CI = [CI1, CI2], wherein CI1 represents a left endpoint of the confidence interval, CI2 represents a right endpoint of the confidence interval, and [CI1, CI2] represents an interval constituted by the two endpoints.
15. The automatic review method of example 14, characterized in that D1 is determined based on a standard deviation of glycated hemoglobin of a sample library and a preset confidence level; and/or, D2 is determined based on a standard deviation of glycated hemoglobin of a sample library and a preset confidence level, wherein the sample library comprises test values of glycated hemoglobin of a plurality of samples; and preferably, D1 and D2 are equal.
16. The automatic review method of any one of examples 1 to 15, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
17. The automatic review method of example 1, characterized in that the method further comprises: prompting a manual review when the basic information of first type, the test value related to blood glucose, and/or the test value related to red blood cell for the subject cannot be obtained.
18. An automatic review method for glycated hemoglobin, characterized in that the method comprises:
   testing a sample of a subject to obtain a test value of glycated hemoglobin for the subject;
   obtaining basic information for the subject;
   obtaining a test value related to blood glucose for the subject and a test value related to red blood cell for the subject;
   calculating an estimated value of glycated hemoglobin for the subject based on the basic information, the test value related to blood glucose and the test value related to red blood cell; and
   performing an automatic review on the test value of glycated hemoglobin for the subject based on the estimated value of glycated hemoglobin for the subject.
19. The automatic review method of example 18, characterized in that the basic information comprises at least one of followings: age, height, weight, body mass index, history of diabetes, medication status, and sample source department.
20. The automatic review method of example 18 or 19, characterized in that calculating an estimated value of glycated hemoglobin for the subject based on the basic information, the test value related to blood glucose and the test value related to red blood cell, comprises:
   inputting the basic information, the test value related to blood glucose and the test value related to red blood cell into a glycated hemoglobin estimation model, so as to obtain the estimated value of glycated hemoglobin for the subject through calculation, wherein the glycated hemoglobin estimation model is configured to use the basic information, the test value related to blood glucose and the test value related to red blood cell as input data, and perform calculation to output the estimated value of glycated hemoglobin for the subject; the glycated hemoglobin estimation model is obtained through training by using a training set, wherein data of the training set comprises first data and second data, the first data is labeled with the second data, the first data comprises basic information, test values related to blood glucose and test values related to red blood cell for other subjects, and the second data comprises test values of glycated hemoglobin for said subjects;
      or
   obtaining a function relationship between the basic information, the test value related to blood glucose, the test value related to red blood cell, and the estimated value of glycated hemoglobin; and obtaining the estimated value of glycated hemoglobin for the subject based on the function relationship, the basic information for the subject, the test value related to blood glucose for the subject and the test value related to red blood cell for the subject.
21. The automatic review method of example 20, characterized in that the function relationship comprises a first function formula; obtaining the estimated value of glycated hemoglobin for the subject based on the function relationship, the basic information for the subject, the test value related to blood glucose for the subject and the test value related to red blood cell for the subject, comprises:
   inputting the basic information for the subject, the test value related to blood glucose for the subject, and the test value related to red blood cell for the subject as input values into the first function formula, so as to obtain the estimated value of glycated hemoglobin for the subject through calculation.
22. The automatic review method of example 21, characterized in that the first function formula comprises a function formula that preforms linear weighting on a plurality of input values.
23. The automatic review method of any one of examples 20 to 22, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
24. An automatic review method for glycated hemoglobin, characterized in that the method comprises:
   testing a sample of a subject to obtain a test value of glycated hemoglobin for the subject;
   obtaining basic information of first type for the subject, wherein the basic information of first type is quantifiable information that represents personal characteristic of the subject;
   obtaining an in vitro diagnostic test value associated with glycated hemoglobin for the subject;
   calculating an estimated value of glycated hemoglobin for the subject based on the basic information of first type and the in vitro diagnostic test value associated with glycated hemoglobin; and
   performing an automatic review on the test value of glycated hemoglobin for the subject based on the estimated value of glycated hemoglobin for the subject.
25. The automatic review method of example 24, characterized in that the in vitro diagnostic test value associated with glycated hemoglobin comprises at least one of followings: a test value related to blood glucose, a test value related to red blood cell.
26. The automatic review method of example 25, characterized in that the test value related to blood glucose comprises: a test value that reflects a related substance involved in blood glucose metabolism and/or a test value that reflects a substance related to blood glucose concentration;
   preferably, the test value that reflects the related substance involved in blood glucose metabolism comprises at least one of followings: a related test value that reflects insulin, and a related test value that reflects a metabolite of insulin; preferably, the related test value that reflects insulin comprises at least one of followings: a test value of glutamate decarboxylase antibody, a test value of insulin autoantibody, a test value of islet cell antibody, a test value of islet cell antigen 2 antibody, and a test value of zinc transporter 8 antibody; the related test value that reflects metabolite of insulin comprises at least one of followings: a test value of serum C-peptide, a test value of adiponectin, and a test value of fetuin;
   preferably, the test value that reflects the substance related to blood glucose concentration comprises at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin; preferably, the test value of blood glucose comprises at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin comprises at least one of followings: a test value of total bilirubin, a test value of direct bilirubin, and a test value of indirect bilirubin;
      and/or
   the test value related to red blood cell comprises at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width.
27. The automatic review method of example 24, characterized in that the in vitro diagnostic test value associated with glycated hemoglobin comprises at least one of followings: a biochemical test value associated with glycated hemoglobin, a blood routine test value associated with glycated hemoglobin, an immunological test value associated with glycated hemoglobin, and a mass spectrometry test value associated with glycated hemoglobin.
28. The automatic review method of example 27, characterized in that the biochemical test value associated with glycated hemoglobin comprises at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, and a test value of bilirubin; preferably, the test value of blood glucose comprises at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin comprises at least one of followings: a test value of total bilirubin, a test value of direct bilirubin, and a test value of indirect bilirubin;
   and/or
   the blood routine test value associated with glycated hemoglobin comprises at least one of followings: a test value of lactic acid, and a test value related to red blood cell; preferably, the test value related to red blood cell comprises at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width;
      and/or
   the immunological test value associated with glycated hemoglobin comprises at least one of followings: a test value of glutamate decarboxylase antibody, a test value of insulin autoantibody, a test value of islet cell antibody, a test value of islet cell antigen 2 antibody, a test value of zinc transporter 8 antibody, a test value of serum C-peptide, a test value of adiponectin, and a test value of fetuin;
      and/or
   the mass spectrometry test value associated with glycated hemoglobin comprises at least one of followings: a test value of 1-anhydroglucitol, and a test value of 5-anhydroglucitol.
29. The automatic review method of any one of examples 25 to 28, characterized in that calculating an estimated value of glycated hemoglobin for the subject based on the basic information of first type and the in vitro diagnostic test value associated with glycated hemoglobin, comprises:
   inputting the basic information of first type and the in vitro diagnostic test value associated with glycated hemoglobin into a glycated hemoglobin estimation model, so as to obtain the estimated value of glycated hemoglobin for the subject through calculation, wherein the glycated hemoglobin estimation model is configured to use the basic information of first type and the in vitro diagnostic test value associated with glycated hemoglobin as input data, and perform calculation to output the estimated value of glycated hemoglobin for the subject; the glycated hemoglobin estimation model is obtained through training by using a training set, wherein data of the training set comprises first data and second data, the first data is labeled with the second data, the first data comprises first-type basic information and in vitro diagnostic test values associated with glycated hemoglobin for other subjects, and the second data comprises test values of glycated hemoglobin for said subjects;
      or
   obtaining a function relationship between the basic information of first type, the in vitro diagnostic test value associated with glycated hemoglobin, and the estimated value of glycated hemoglobin; obtaining the estimated value of glycated hemoglobin for the subject based on the function relationship, the basic information of first type for the subject and the in vitro diagnostic test value associated with glycated hemoglobin for the subject.
30. The automatic review method of example 29, characterized in that the function relationship comprises a first function formula; obtaining the estimated value of glycated hemoglobin for the subject based on the function relationship, the basic information of first type for the subject and the in vitro diagnostic test value associated with glycated hemoglobin for the subject, comprises:
   inputting the basic information of first type for the subject and the in vitro diagnostic test value associated with glycated hemoglobin for the subject as input values into the first function formula, so as to obtain the estimated value of glycated hemoglobin for the subject through calculation.
31. The automatic review method of any one of examples 24 to 30, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
32. An automatic review method for glycated hemoglobin, characterized in that the method comprises:
   testing a sample of a subject to obtain a test value of glycated hemoglobin for the subject;
   obtaining basic information for the subject;
   obtaining an in vitro diagnostic test value associated with glycated hemoglobin for the subject;
   calculating an estimated value of glycated hemoglobin for the subject based on the basic information and the in vitro diagnostic test value associated with glycated hemoglobin; and
   performing an automatic review on the test value of glycated hemoglobin for the subject based on the estimated value of glycated hemoglobin for the subject.
33. The automatic review method of example 32, characterized in that calculating an estimated value of glycated hemoglobin for the subject based on the basic information and the in vitro diagnostic test value associated with glycated hemoglobin, comprises:
   inputting the basic information and the in vitro diagnostic test value associated with glycated hemoglobin into a glycated hemoglobin estimation model, so as to obtain the estimated value of glycated hemoglobin for the subject through calculation, wherein the glycated hemoglobin estimation model is configured to use the basic information and the in vitro diagnostic test value associated with glycated hemoglobin as input data, and perform calculation to output the estimated value of glycated hemoglobin for the subject; the glycated hemoglobin estimation model is obtained through training by using a training set, wherein data of the training set comprises first data and second data, the first data is labeled with the second data, the first data comprises basic information and in vitro diagnostic test values associated with glycated hemoglobin for other subjects, and the second data comprises test values of glycated hemoglobin for said subjects;
      or
   obtaining a function relationship between the basic information, the in vitro diagnostic test value associated with glycated hemoglobin, and the estimated value of glycated hemoglobin; obtaining the estimated value of glycated hemoglobin for the subject based on the function relationship, the basic information and the in vitro diagnostic test value associated with glycated hemoglobin.
34. The automatic review method of example 33, characterized in that the function relationship comprises a first function formula; obtaining the estimated value of glycated hemoglobin for the subject based on the function relationship, the basic information and the in vitro diagnostic test value associated with glycated hemoglobin, comprises:
   inputting the basic information for the subject, and the in vitro diagnostic test value associated with glycated hemoglobin for the subject as input values into the first function formula, so as to obtain the estimated value of glycated hemoglobin for the subject through calculation.
35. The automatic review method of any one of examples 32 to 34, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
36. An automatic review method for glycated hemoglobin, characterized in that the method comprises:
   testing a sample of a subject to obtain a test value of glycated hemoglobin for the subject;
   obtaining a confidence interval of glycated hemoglobin for the subject, wherein the confidence interval of glycated hemoglobin for the subject is configured to evaluate the test value of glycated hemoglobin for the subject; and
   performing an automatic review on the test value of glycated hemoglobin for the subject based on the confidence interval of glycated hemoglobin of the subject.
37. The automatic review method of example 36, characterized in that the confidence interval of glycated hemoglobin for the subject is obtained through calculation based on an estimated value of glycated hemoglobin for the subject; or, the confidence interval of glycated hemoglobin for the subject is obtained through calculation based on a mean and a standard deviation of test values of glycated hemoglobin of a plurality of samples in a sample library.
38. The automatic review method of example 37, characterized in that the estimated value of glycated hemoglobin for the subject is obtained through calculation based on basic information and an in vitro diagnostic test value associated with glycated hemoglobin for the subject.
39. The automatic review method of any one of examples 36 to 38, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
40. An automatic review method for glycated hemoglobin, characterized in that the method comprises:
   testing a sample of a subject to obtain a test value of glycated hemoglobin for the subject;
   obtaining information of the subject, wherein the information comprises basic information for the subject or an in vitro diagnostic test value associated with glycated hemoglobin for the subject;
   calculating an estimated value of glycated hemoglobin for the subject based on the information of the subject; and
   performing an automatic review on the test value of glycated hemoglobin for the subject based on the estimated value of glycated hemoglobin for the subject.
41. The automatic review method of example 40, characterized in that calculating an estimated value of glycated hemoglobin for the subject based on the information of the subject, comprises:
   inputting the information of the subject into a glycated hemoglobin estimation model, so as to obtain the estimated value of glycated hemoglobin for the subject through calculation, wherein the glycated hemoglobin estimation model is configured to use the information of the subject as input data, and perform calculation to output the estimated value of glycated hemoglobin for the subject; the glycated hemoglobin estimation model is obtained through training by using a training set, wherein data of the training set comprises first data and second data, the first data is labeled with the second data, the first data comprise information of other subjects, and the second data comprises test values of glycated hemoglobin for said subjects;
      or
   obtaining a function relationship between the information and the estimated value of glycated hemoglobin; and obtaining the estimated value of glycated hemoglobin for the subject based on the function relationship and the information of the subject.
42. The automatic review method of example 41, characterized in that the function relationship comprises a first function formula; obtaining the estimated value of glycated hemoglobin for the subject based on the function relationship and the information of the subject, comprises:
   inputting the information of the subject as an input value into the first function formula, so as to obtain the estimated value of glycated hemoglobin for the subject through calculation.
43. The automatic review method of any one of examples 40 to 42, characterized in that the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
44. A sample analysis device, characterized in that the device comprises:
   a measurement component configured to test a sample to obtain a test value for an item;
   a sample component configured to provide a sample to the measurement component;
   a reagent component configured to provide a reagent to the measurement component; and
   a processor configured to perform the method of any one of examples 1 to 43.
45. A sample analysis system, characterized in that the system comprises: a processor and a plurality of cascaded analysis devices, wherein each of the analysis devices is configured to test a sample to obtain a test value for an item, and the processor is configured to perform the method of any one of examples 1 to 43.

## Claims

1. An automatic review method for glycated hemoglobin, **characterized in that** the method comprises:
testing a sample of a subject to obtain a test value of glycated hemoglobin for the subject;
obtaining information of the subject, wherein the information comprises basic information for the subject and/or an in vitro diagnostic test value associated with glycated hemoglobin for the subject;
calculating an estimated value of glycated hemoglobin for the subject based on the information of the subject; and
performing an automatic review on the test value of glycated hemoglobin for the subject based on the estimated value of glycated hemoglobin for the subject.

2. The automatic review method of claim 1, **characterized in that** the basic information comprises at least one of followings: age, height, weight, body mass index, history of diabetes, medication status, and sample source department; and/or
the in vitro diagnostic test value associated with glycated hemoglobin comprises at least one of followings: a biochemical test value associated with glycated hemoglobin, a blood routine test value associated with glycated hemoglobin, an immunological test value associated with glycated hemoglobin, and a mass spectrometry test value associated with glycated hemoglobin.

3. The automatic review method of claim 1 or 2, **characterized in that** the information comprises:
basic information of first type for the subject, wherein the basic information of first type is quantifiable information that represents personal characteristic of the subject; and/or
a test value related to blood glucose for the subject and a test value related to red blood cell for the subject.

4. The automatic review method of claim 3, **characterized in that** the test value related to blood glucose comprises: a test value that reflects a related substance involved in blood glucose metabolism and/or a test value that reflects a substance related to blood glucose concentration;
preferably, the test value that reflects the related substance involved in blood glucose metabolism comprises at least one of followings: a related test value that reflects insulin, and a related test value that reflects a metabolite of insulin; preferably, the related test value that reflects insulin comprises at least one of followings: a test value of glutamate decarboxylase antibody, a test value of insulin autoantibody, a test value of islet cell antibody, a test value of islet cell antigen 2 antibody, and a test value of zinc transporter 8 antibody; the related test value that reflects the metabolite of insulin comprises at least one of followings: a test value of serum C-peptide, a test value of adiponectin, and a test value of fetuin;
preferably, the test value that reflects the substance related to blood glucose concentration comprises at least one of followings: a test value of blood glucose, a test value of 1-anhydroglucitol, a test value of 5-anhydroglucitol, a test value of lactic acid, a test value of glycated serum protein, a test value of fructosamine, a test value of glycated serum albumin, a test value of advanced glycation end products, and a test value of bilirubin; preferably, the test value of blood glucose comprises at least one of followings: a test value of fasting blood glucose, a test value of 1-hour postprandial blood glucose, a test value of 2-hour postprandial blood glucose, and a test value of 3-hour postprandial blood glucose; preferably, the test value of bilirubin comprises at least one of followings: a test value of total bilirubin, a test value of direct bilirubin, and a test value of indirect bilirubin; and/or
the test value related to red blood cell comprises at least one of followings: a test value of red blood cell count, a test value of hemoglobin content, a test value of mean corpuscular volume, a test value of mean corpuscular hemoglobin, a test value of mean corpuscular hemoglobin concentration, a test value of hematocrit, and a test value of red blood cell distribution width; and/or
the basic information of first type comprises at least one of followings: age, height, weight, and body mass index.

5. The automatic review method of any one of claims 1 to 4, **characterized in that** calculating an estimated value of glycated hemoglobin for the subject based on the information of the subject, comprises:
inputting the information of the subject into a glycated hemoglobin estimation model, so as to obtain the estimated value of glycated hemoglobin for the subject through calculation, wherein the glycated hemoglobin estimation model is configured to use the information of the subject as input data, and perform calculation to output the estimated value of glycated hemoglobin for the subject; the glycated hemoglobin estimation model is obtained through training by using a training set, wherein data of the training set comprises first data and second data, the first data is labeled with the second data, the first data comprise information of other subjects, and the second data comprises test values of glycated hemoglobin for said subjects;
preferably, inputting the basic information of first type, the test value related to blood glucose and the test value related to red blood cell into a glycated hemoglobin estimation model, so as to obtain the estimated value of glycated hemoglobin for the subject through calculation, wherein the glycated hemoglobin estimation model is configured to use the basic information of first type, the test value related to blood glucose and the test value related to red blood cell as input data, and perform calculation to output the estimated value of glycated hemoglobin for the subject;
wherein the glycated hemoglobin estimation model is obtained through training by using a training set, wherein data of the training set comprises first data and second data, the first data is labeled with the second data, the first data comprises first-type basic information, test values related to blood glucose and test values related to red blood cell for other subjects, and the second data comprises test values of glycated hemoglobin for said subjects.

6. The automatic review method of claim 5, **characterized in that** the glycated hemoglobin estimation model comprises: a convolutional neural network-based model, a recurrent neural network-based model, a generative adversarial neural network-based model, an attention neural network-based model, or a fully connected network-based model.

7. The automatic review method of any one of claims 1 to 4, **characterized in that** calculating an estimated value of glycated hemoglobin for the subject based on the information of the subject, comprises:
obtaining a function relationship between the information and the estimated value of glycated hemoglobin; and obtaining the estimated value of glycated hemoglobin for the subject based on the function relationship and the information of the subject;
preferably, obtaining a function relationship between the basic information of first type, the test value related to blood glucose, the test value related to red blood cell, and the estimated value of glycated hemoglobin; and
obtaining the estimated value of glycated hemoglobin for the subject based on the function relationship, the basic information of first type for the subject, the test value related to blood glucose for the subject, and the test value related to red blood cell for the subject.

8. The automatic review method of claim 7, **characterized in that** the function relationship comprises a first function formula, preferably, the first function formula comprises a function formula that preforms linear weighting on a plurality of input values;
obtaining the estimated value of glycated hemoglobin for the subject based on the function relationship and the information of the subject, comprises:
inputting the information of the subject as an input value into the first function formula, so as to obtain the estimated value of glycated hemoglobin for the subject through calculation;
preferably, inputting the basic information of first type for the subject, the test value related to blood glucose for the subject, and the test value related to red blood cell for the subject as input values into the first function formula, so as to obtain the estimated value of glycated hemoglobin for the subject through calculation.

9. The automatic review method of claim 8, **characterized in that** the basic information of first type is age, the test value related to blood glucose is a test value of blood glucose, the test value related to red blood cell is a test value of red blood cell count, and the first function formula is: HbA1c' = a1 + a2*Age + a3*GLU + a4*RBC, wherein HbA1c' represents the estimated value of glycated hemoglobin, Age represents the age, GLU represents the test value of blood glucose, RBC represents the test value of red blood cell count, and a1, a2, a3, and a4 represent constants; preferably, a1 ranges from 1.6 to 2.6, a2 ranges from 0.01 to 0.02, a3 ranges from 0.4 to 0.5, and a4 ranges from 0.07 to 0.24; or
the basic information of first type is age, the test value related to blood glucose comprises a test value of fasting blood glucose, a test value of 2-hour postprandial blood glucose and a test value of bilirubin, the test value related to red blood cell is a test value of red blood cell count, and the first function formula is: HbA1c' = b1 + b2*Age + b3*GLU 1 + b4*G120 + b5/IB² + b6*RBC, wherein HbA1c' represents the estimated value of glycated hemoglobin, Age represents the age, GLU1 represents the test value of fasting blood glucose, G120 represents the test value of 2-hour postprandial blood glucose, IB represents the test value of bilirubin, RBC represents the test value of red blood cell count, and b1, b2, b3, b4, b5, and b6 represent constants; preferably, b1 ranges from 1.6 to 2.5, b2 ranges from 0.001 to 0.009, b3 ranges from 0.24 to 0.3, b4 ranges from 0.09 to 0.13, b5 ranges from 2.5 to 10.9, and b6 ranges from 0.13 to 0.29; or
the basic information of first type is age, the test value related to blood glucose comprises a test value of blood glucose and a test value of bilirubin, the test value related to red blood cell is a test value of red blood cell count, and the first function formula is: HbA1c' = c1 + c2*Age + c3*GLU + c4/IB² + c5*RBC, wherein HbA1c' represents the estimated value of glycated hemoglobin, Age represents the age, GLU represents the test value of blood glucose, IB represents the test value of bilirubin, and RBC represents the test value of red blood cell count, and c1, c2, c3, c4, and c5 represent constants; preferably, c1 ranges from 1.2 to 2.3, c2 ranges from 0.01 to 0.02, c3 ranges from 0.4 to 0.47, c4 ranges from 2.8 to 12.97, and c5 ranges from 0.1 to 0.29.

10. The automatic review method of any one of claims 1 to 9, **characterized in that** performing an automatic review on the test value of glycated hemoglobin for the subject based on the estimated value of glycated hemoglobin for the subject, comprises: calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate.

11. The automatic review method of claim 10, **characterized in that** calculating a matching degree between the estimated value and the test value of glycated hemoglobin for the subject, and performing the automatic review on the test value of glycated hemoglobin for the subject based on the matching degree to determine whether the test value of glycated hemoglobin for the subject is accurate, comprises:
calculating a confidence interval of glycated hemoglobin for the subject based on the estimated value of glycated hemoglobin for the subject, preferably, obtaining the confidence interval of glycated hemoglobin for the subject through calculation based on the estimated value of glycated hemoglobin for the subject and a second function formula; and
determining that the test value of glycated hemoglobin for the subject is accurate when the test value of glycated hemoglobin for the subject falls within the confidence interval of glycated hemoglobin for the subject.

12. The automatic review method of claim 11, **characterized in that** the second function formula comprises: CI1 = HbA1c' - D1; CI2 = HbA1c' + D2, wherein HbA1c' represents the estimated value of glycated hemoglobin, and D1 and D2 represent constants;
the confidence interval CI of glycated hemoglobin for the subject satisfies: CI = [CI1, CI2], wherein CI1 represents a left endpoint of the confidence interval, CI2 represents a right endpoint of the confidence interval, and [CI1, CI2] represents an interval constituted by the two endpoints;
preferably, D1 and D2 are equal, and both D1 and D2 are determined based on a standard deviation of glycated hemoglobin of a sample library and a preset confidence level, wherein the sample library comprises test values of glycated hemoglobin of a plurality of samples.

13. The automatic review method of any one of claims 1 to 12, **characterized in that** the automatic review comprises: outputting the test value of glycated hemoglobin for the subject when the test value and the estimated value of glycated hemoglobin for the subject satisfy a preset relationship; or prompting a manual review when the test value and the estimated value of glycated hemoglobin for the subject do not satisfy the preset relationship.
preferably, the method further comprises: prompting a manual review when the basic information of first type, the test value related to blood glucose, and/or the test value related to red blood cell for the subject cannot be obtained.

14. A sample analysis device, **characterized in that** the device comprises:
a measurement component configured to test a sample to obtain a test value for an item;
a sample component configured to provide a sample to the measurement component;
a reagent component configured to provide a reagent to the measurement component; and
a processor configured to perform the method of any one of claims 1 to 13.

15. A sample analysis system, **characterized in that** the system comprises: a processor and a plurality of cascaded analysis devices, wherein each of the analysis devices is configured to test a sample to obtain a test value for an item, and the processor is configured to perform the method of any one of claims 1 to 13.
